# EUROPEAN PATENT SPECIFICATION

(11) **EP 0 468 042 B1**
(45) Date of publication and mention of the grant of the patent: **19.04.1995**
(21) Application number: 91905084.9
(22) Date of filing: 13.02.1991
(51) Int. Cl.: C07C 401/00, A61K 31/59

(54) **HOMOLOGATED VITAMIN D2 COMPOUNDS AND THE CORRESPONDING 1alpha-HYDROXYLATED DERIVATIVES**
HOMOLOGIERTE VITAMIN-D2-VERBINDUNGEN UND KORRESPONDIERENDE 1alpha-HYDROXYLIERTE VERBINDUNGEN
DES COMPOSES DE VITAMINE D2 HOMOLOGUES ET LES DERIVES DE 1alpha-HYDROXYLES CORRESPONDANTS

(30) Priority: 14.02.1990 US 481990
(43) Date of publication of application: 29.01.1992
(73) Proprietor: WISCONSIN ALUMNI RESEARCH FOUNDATION, Madison, WI 53705 (US)
(72) Inventor: DELUCA, Hector, Floyd, Deerfield, WI 53531 (US); SCHNOES, Heinrich, Konstantine, Madison, WI 53705 (US); PERLMAN, Kato, Leonard, Madison, WI 53711 (US)
(74) Representative: Ellis-Jones, Patrick George Armine
(86) International application number: PCT/US91/00974
(87) International publication number: WO 91/12239

(56) References cited:
- EP-A- 0 386 793
- EP-A- 0 402 982
- EP-A- 0 421 561
- WO-A-85/03939
- WO-A-85/05622
- WO-A-86/02078
- WO-A-86/04333
- WO-A-89/10351
- WO-A-89/10352
- WO-A-91/00271
- FR-A- 2 554 444
- GB-A- 2 139 627
- US-A- 4 847 012
- CHEMICAL AND PHARMACEUTICAL BULLETIN, vol. 34, no. 11, November 1986, The Pharmaceutical Society of Japan; H. SAI et al, "Synthesis of some side-chain homologues of 1alpha;25-dihydroxy-vitamin D3 and investigation of their biological activities", pages 4508-4515
- CHEMICAL ABSTRACTS, vol. 101, no. 15, 8 October 1984, Columbus, Ohio, US; I. A. ALEKSEEVA et al, "Activity of 5,6-trans- and 22-dehydro analogs of 1,25-dihydroxyvitamin D3", page 601, abstract no. 129315q

## Description

This invention relates to biologically active vitamin D compounds.

More specifically, this invention relates to novel 24, 26 and/or 27 homalogated derivatives of vitamin D₂ and the corresponding hydroxylated forms thereof.

### Background of the Invention

The D vitamins are very important agents for the control of calcium and phosphate metabolism in animals and humans, and have long been used as dietary supplements and in clinical practice to assure proper bone growth and development. It is now known that the in vivo activity of these vitamins, specifically of vitamin D₂ and D₃, is dependent on metabolism to hydroxylated forms. Thus, vitamin D₃ undergoes two successive hydroxylation reactions in vivo, leading first to 25-hydroxyvitamin D₃ and then to 1,25-dihydroxyvitamin D₃ and the latter is thought to be the compound responsible for the well-known beneficial effects of vitamin D₃. Likewise, vitamin D₂, which is commonly used as a dietary supplement, undergoes an analogous hydroxylation sequence to its active forms, being first converted to 25-hydroxyvitamin D₂ (25-OH-D₂) and then to 1,25-dihydroxyvitamin D₂ (1,25-(OH)₂D₂). These facts are well established and well known in the art [see, for example, Suda et al. Biochemistry 8, 3515 (1969) and Jones et al. Biochemistry 14, 1250 (1975)].

Like the metabolites of the vitamin D₃ series, the hydroxylated forms of vitamin D₂ named above are, because of their potency and other beneficial properties, highly desirable dietary supplements, or pharmaceutical agents, for the cure or prevention of bone or related diseases, and their value and possible use is recognized in patents relating to these compounds [U. S. Letters Pat. Nos. 3,585,221 and 3,880,894].

Whereas many metabolites of vitamin D₃ have been prepared by chemical synthesis, there has been less work on the preparation of vitamin D₂ metabolites. The known synthetic processes for the metabolites of the D₃-series (especially as far as they relate to the preparation of side chain hydroxylated compounds) are, of course, in general not suitable for the preparation of the corresponding vitamin D₂ metabolites, since the latter are characterized by a side chain structure (i.e. presence of a double bond and an extra methyl group) which requires a different synthetic approach from that applicable to side chain hydroxylated D₃ compounds.

Various approaches for the preparation of vitamin D₂ metabolites are known, and are described in U. S. Patent Nos. 4,448,721, 4,847,012 and 4,769,181. Other preparations of 25-OH-D₂ and 1α,25-(OH)₂D₂ compounds involving condensation of side chains with a steroid nucleus are shown in Yamada et al, "Facile And Stereoselective Synthesis of 25-hydroxyvitamin D₂", Tetrahedron Letters, Vol. 25, No. 33, pp. 3347-3350, 1984 and in Tsuji et al, "A New And Convenient Synthesis of 1α,25-Dihydroxyvitamin D₂ And Its 24R-Epimer", Bull, Chem. Soc. Jpn., Vol. 62, No. 10, pp. 3132-3137, 1989. Perlman et al have reported the preparation of the epimer of 1α-OH-D₂ by condensation of a suitable side chain fragment with a vitamin D nucleus in J. Chem. Soc. Chem. Com. pp. 1113-1115, 1989.

The natural vitamin D-derived hormone, 1α,25-dihydroxyvitamin D₃, and its 25-deoxy analog, 1α-hydroxyvitamin D₃, both exhibit high activity in vivo, being known as potent stimulators of the intestinal absorption of calcium and the mobilization of calcium from bone and as effective promoters of bone calcification. A very similar activity pattern is shown by 1α,25-dihydroxyvitamin D₂ (U.S. Patent 3,880,894) and its 25-deoxy analog, 1α-hydroxyvitamin D₂ (U.S. Patent 3,907,843). These compounds likewise elicit the full spectrum of vitamin D-type responses such as intestinal calcium transport, bone mineral mobilization and bone calcification response in the animal or human. Structurally, 1α,25-dihydroxyvitamin D₂ and 1α-hydroxyvitamin D₂ are characterized by having a C-24 stereochemistry as it occurs in the side chain of ergosterol, i.e. these compounds are defined by the structures shown below, where R represents side chains (a) and (b), respectively: 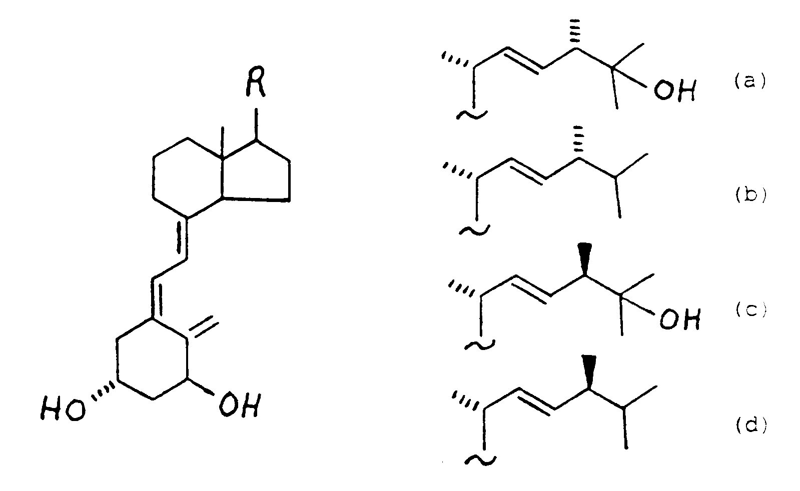
More recently the C-24-epimer of 1α,25-dihydroxyvitamin D₂ and 1α-hydroxyvitamin D₂ has been prepared and tested. These compounds are characterized by the structures shown above, where R represents side chains (c) and (d) respectively. Remarkably these C-24-epimeric vitamin D derivatives exhibit a distinctly different biological activity in that they promote intestinal calcium absorption and the calcification of bone, but elicit little or no bone calcium mobilization response.

### Disclosure of Invention

This invention provides new homologated vitamin D₂ analogs which may be represented by the structure below, as well as the 1α-hydroxy-analogs and the protected hydroxy derivatives of the compounds: 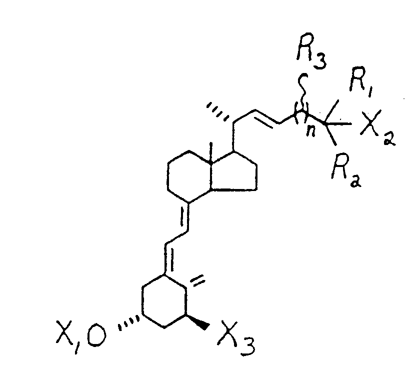
where the configuration about carbon 24 may be R or S and wherein n is an integer having a value of from 1 to 5, X₁ is selected from hydrogen or a hydroxy protecting group, X₂ is selected from hydrogen, hydroxy, and protected hydroxy, X₃ is selected from hydrogen, hydroxy, and protected hydroxy, R₃ is selected from alkyl, hydroxy, protected hydroxy, hydrogen or fluorine, and wherein R₁ and R₂, which may be the same or different, are each selected from an alkyl or aryl group, with the proviso that when X₁ is hydrogen, X₂ is hydrogen or hydroxy, X₃ is hydrogen or hydroxy and n is 1, R₁ and R₂ cannot both be methyl and R₃ cannot be hydrogen or methyl.

These compounds are distinguished from the previously known 1α-hydroxyvitamin D₂ and 1α-hydroxy-24-epi-vitamin D₂ compounds by homologation at the 24, 26 and/or 27 positions. Specific compounds disclosed include 26,27-dihomo-1α-hydroxyvitamin D₂, 26,27-dihomo-1α,25-dihydroxyvitamin D₂, 26,27-dihomo-1α-hydroxy-24-epi-vitamin D₂, 26,27-dihomo-1α, 25-dihydroxy-24-epi-vitamin D₂, and the corresponding 26,27-tetrahomo compounds, as well as 24-dihomo-1α-hydroxy-24-epi-vitamin D₂, 24-dihomo-1α,25-dihydroxy-24-epi-vitamin D₂, and the corresponding 24-trihomo compounds. It should be noted that with respect to all homologated compounds i.e. whether the compound is 24 and/or 26 and/or 27 homologated, the above-noted structural formula encompasses 25-hydroxylated compounds, 1α-hydroxylated compounds, as well as 1α,25-dihydroxylated compounds.

It should be noted in this description that the term "24-dihomo" refers to the addition of two methylene groups substituted with the group R₃ at the carbon 24 position in the side chain (so that n is 3). Likewise, the term "24-trihomo" refers to the addition of three such substituted methylene groups (so that n is 4). Also, the term "26,27-dihomo" refers to the addition of a methyl group at the carbon 26 and 27 positions so that R₁ and R₂ are ethyl groups. Likewise, the term "26,27-tetrahomo" refers to the addition of an ethyl group at the 26 and 27 positions so that R₁ and R₂ are propyl groups.

A novel and convenient synthesis of vitamin D₂ compounds has now been developed and is also described herein. This synthesis provides homologated vitamin D₂ compounds characterized by the structures previously shown as well as non-homologated vitamin D₂ compounds shown below where X₂ is hydrogen, such as vitamin D₂ itself and the 24-epimer of vitamin D₂, namely 24-epi-vitamin D₂ (24-epi D₂), characterized by the structures shown below where X₁ and X₂ are both hydrogen and R₁, R₂ and R₃ are methyl. This synthesis also provides 25 hydroxylated vitamin D₂ compounds, such as 25-hydroxyvitamin D₂ (25-OH-D₂) and the 24-epimer of 25-hydroxyvitamin D₂, namely 25-hydroxy-24-epi-vitamin D₂ (25-OH-24-epi D₂), characterized by the structures shown below where X₁ is hydrogen, X₂ is hydroxy and R₁, R₂ and R₃ are methyl. 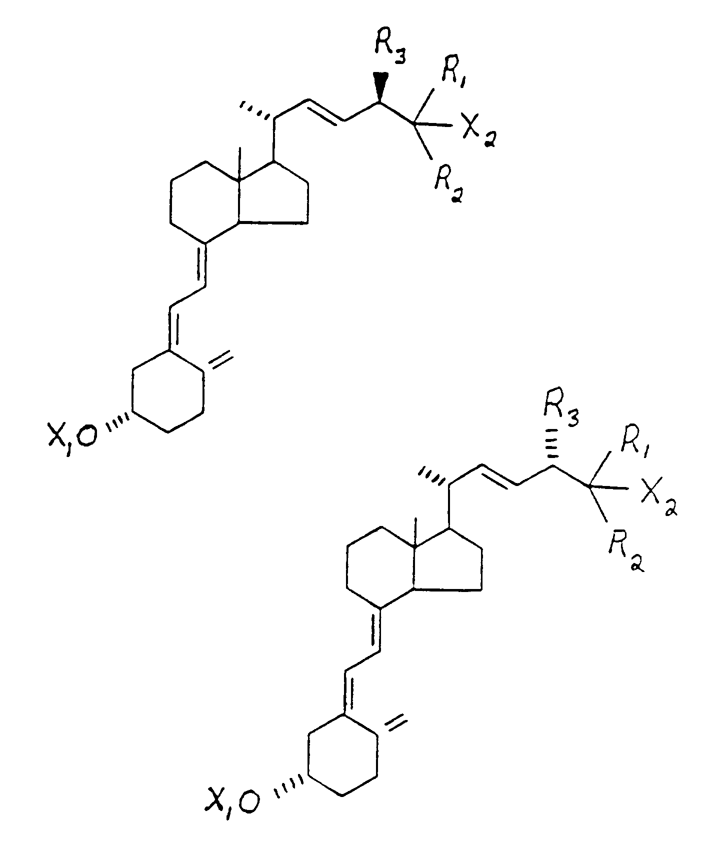
This synthesis thus provides compounds where X₂ may be either hydrogen or hydroxy, as well as the corresponding alkyl or aryl analogs thereof, characterized by the structure above where R₁ and R₂ are alkyl or aryl, and the corresponding side chain substituted derivatives there R₃ is alkyl, hydroxy, protected hydroxy (as defined below for X₁ and X₂), hydrogen or fluorine, and the hydroxy-protected derivatives of these compounds characterized by the structures above, where X₁ is selected from the group consisting of acyl, alkylsilyl, or alkoxyalkyl and X₂ is selected from the group consisting of O-acyl, O-alkylsilyl, or O-alkoxyalkyl.

In addition, the present process provides the 5,6-trans-isomers of the above compounds, as well as the 24 and/or 26 and/or 27 homologated compounds previously shown herein. Furthermore, the above compounds can be 1α-hydroxylated by known methods, so as to produce the corresponding 1α-hydroxyvitamin D derivatives. Especially preferred examples of the latter are 1α-hydroxyvitamin D₂, 1α,25-dihydroxyvitamin D₂, 1α-24-epi-vitamin D₂ and 1α,25-dihydroxy-24-epi-vitamin D₂.

The term "acyl", as used in this specification or in the claims, signifies an aliphatic acyl group of from 1 to about 6 carbons, in all possible isomeric forms (e.g. formyl, acetyl, propionyl, butyryl, isobutyryl, valeryl, etc.), or an aromatic acyl group (aroyl group) such as benzoyl, the isomeric methtlbenzoyls, the isomeric nitro- or halo-benzoyls, etc., or a dicarboxylic acyl group of from 2 to 6 atoms chain length, i.e. acyl groups of the type ROOC(CH₂)ₙCO-, or ROCCH₂-O-CH₂CO-, where n has values between 0 and 4 inclusive and R is hydrogen or an alkyl radical, such as oxalyl, malonyl, succinoyl, glutaryl, adipyl, diglycolyl. The term "alkyl" refers to a lower alkyl group of 1 to 6 carbons in all possible isomeric forms, e.g. methyl, ethyl, propyl, isopropyl, butyl, isobutyl, sec.-butyl, pentyl, etc., and the work "aryl" signifies a phenyl or substituted phenyl group, e.g. alkylphenyl, methoxyphenyl, etc. The term "alkylsilyl" refers to trialkyl silicone groupings where the alkyl groups may be the same or different as exemplified by trimethylsilyl, triethylsilyl, dimethyl-tert.-butylsilyl and similar groupings. The term "alkoxyalkyl" refers to protecting groups such as methoxymethyl, ethoxymethyl, methoxyethoxymethyl, and similar alkoxymethyl groups, as well as the related cyclic structures, such as tetrahydropyranyl or tetrahydrofuranyl.

The overall process developed for the preparation of the above compounds may be divided into two general phases, namely (a) addition of a completed, preformed side chain fragment to a suitable steroidal precursor to produce a 5,7-diene steroid as the central intermediate, and (b) conversion of this 5,7-diene to the vitamin D structure to produce the desired vitamin D compound, and, if desired, (c) further conversion of the latter product to the corresponding 1α-hydroxyvitamin D compound. This process avoids the relatively difficult step of isomer separation which is required after conversion in the process of U. S. patent No. 4,448,721. The process of the present invention also increases the yield of the end product since it utilizes a completed, preformed pure isomer side chain fragment to make the desired end product, rather than a mixture of side chain isomers as in U. S. Patent No. 4,448,721.

In general terms, the process of this invention comprises the reaction of a steroidal 22-aldehyde of the structure: 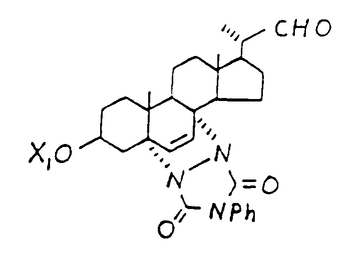
where X₁ is hydrogen or a hydroxy-protecting group, with a sulfone derivative of the general formula,

ArSO₂CH₂R

where Ar represents a phenyl or tolyl group, and R is selected from the group consisting of straight or branched, substituted or unsubstituted, hydrocarbon radicals of from 1 to 25 carbon atoms, where the substituents are selected from the group consisting of hydroxy, protected hydroxy, and fluorine.

The coupling reaction, conducted in a basic medium, between the above aldehyde and sulfone derivatives, yields a condensation product of the formula: 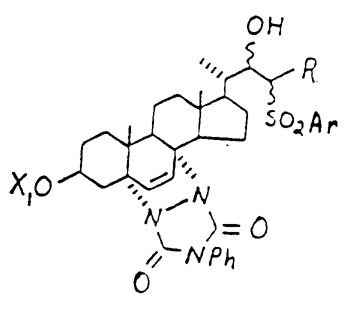
which is then subjected to reduction (either as the 22-hydroxy, or as the corresponding 22-O-acylated derivative) using metal amalgams (Na, Al, Zn amalgams) or related dissolving metal reduction systems, so as to provide the 22, 23-unsaturated steroid of the formula: 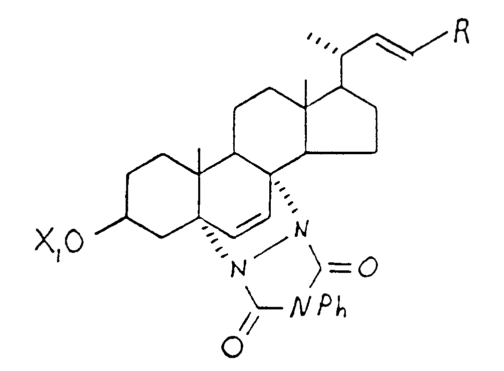
where R and X₁ represent the groupings defined above. This steroid intermediate can then be further converted by known reactions to the desired vitamin D compounds.

It is readily apparent that by suitable variation of R in the aryl sulfone derivative used in the above coupling process a range of different vitamin D compounds can be produced.

### 25-Hydroxylated Compounds (X₂ Is Hydroxy)

The reaction sequence illustrated by Process Scheme I presents a specific embodiment of the overall process, whereas Process Scheme II illustrates preparation of an appropriate side chain unit for addition to the steroid-22-aldehyde as shown in Scheme I.

Starting materials for the present process are steroidal 22-aldehydes, such as, for example, the PTAD-diene-protected-22-aldehyde (4) shown in Scheme I (where PTAD refers to the phenyltriazoline-3,5-dione-protecting group shown), which in turn can be prepared from ergosterol by the known steps (Scheme I).

The first step of this process comprises the addition of a suitable side chain fragment. Thus, condensation of aldehyde (4) with a sulfonyl-side chain fragment as shown in Scheme I (sulfone (21), further described below) present in the form of its anion, in an ether or hydrocarbon solvent, provides the hydroxy-sulfone intermediate (5). The anion of the sulfone (21) side chain fragment is generated by treatment of the sulfone with a strong base, such as lithium diethylamide, n-butyl lithium or methyl or ethyl magnesium bromide (or similar Grignard reagent) in an ether or hydrocarbon solvent, and to this solution of sulfone anion the steroid aldehyde (compound 4) as an ether or hydrocarbon solution is then added. The reaction is best effected under an inert atmosphere.

The next step comprises the removal of the hydroxy-and phenylsulfonyl groups in the side chain with formation of the 22(23)-trans-double bond. Thus, treatment of compound (5), in methanol solution saturated with NaHPO₄, with sodium amalgam under an inert atmosphere, gives compound (6) featuring the desired trans-22-double bond in the side chain. If desired, the 22-hydroxy group in compound (5) can also be acylated or sulfonylated (e.g. mesylated) prior to the Na/Hg-reduction step, but this is not generally required.

It is to be noted that, as shown in Process Scheme I, addition of the side chain fragment, sulfone (21), to the aldehyde (4), does not cause epimerization at the asymmetric center of carbon 20, i.e. the stereochemistry at that center is retained, as is required. If desired, retention of stereochemistry at carbon 20 may be checked at this stage of the synthesis by the conversion of intermediates of type (6) back to the original aldehyde starting materials. For example, subjecting compound (6) to ozonolysis with reductive work-up, using fully conventional and standard conditions, yields the corresponding C-22-aldehyde, i.e. the aldehyde of structure (4). Spectroscopic and chromatographic comparison of the aldehyde obtained from ozonolysis with the original starting material confirms retention of C-20 stereochemistry.

The next operation of the process involves conversion of these ring B-protected steroids to the desired 5,7-diene intermediate (7). In the case of the PTAD-diene-protected compound (6), this conversion is accomplished in a single step, namely treatment of (6) with a strong hydride reducing agent (e.g. LiAlH₄) in an ether solvent at reflux temperature gives the diene (7). The diene (7) is then converted to its 25-hydroxylated form (8) by known procedures in accordance with Scheme I.

Conversion of 5,7-diene (8) to the final vitamin D products (10) or (15) comprises a sequence of several steps. The sequence shown in Process Scheme I involves first the irradiation of an ether or hydrocarbon solution of the 5,7-diene (8) with ultraviolet light to yield the previtamin analog (9) which by warming (50°-90°C) in a suitable solvent (e.g. ethanol, hexane) undergoes isomerization to the 25-hydroxyvitamin D₂ compound (10).

Thereafter, compound (10) may be converted to the 1,25-dihydroxyvitamin D₂ compound (15) by the known steps shown in Scheme I. Reference is made to U. S. Patent Nos. 4,260,549 and 4,554,106 for the prior art relevant to these transformations.

The side chain fragment, sulfone (21), as used in Scheme I is specifically the (R) enantiomer. Therefore, compounds (10) or (15) are obtained as the C-24-R-epimers, 25-hydroxy-24-epi-vitamin D₂ (10) or 1,25-dihydroxy-24-epi-vitamin D₂ (15), respectively. Thus, compound (10) or (15) are prepared in epimerically pure form, and C-24-epimer separation as required in the process disclosed in U. S. Patent No. 4,448,721, is not necessary. Use of the (S)-epimer of sulfone (21) in the present process yields specifically 25-OH-D₂, as well as, of course, the respective 1,25-dihydroxyvitamin D₂ compound.

The 5,7-diene (7) may be used as the free hydroxy compound or as its hydroxy-protected form, where the hydroxy-protecting groups (at C-3 and/or C-25) may be acyl, alkylsilyl or alkoxyalkyl groups as previously defined. Thus, the 25-OH-D₂ product will be obtained as the free hydroxy compound or, if desired, as the C-3, or C-25-hydroxy-protected, or 3,25-dihydroxy-protected derivatives. Synthesis according to Scheme I would provide the 25-OH-D₂ products as the free hydroxy compounds but analogous conversion of 5,7-diene intermediate (7) as the 3-, or 25-protected, or 3,25-di-protected derivative will yield the corresponding hydroxy-protected derivatives of the 25-OH-D₂ products.

The individual 25-OH-D₂ epimers, i.e. 25-OH-D₂ or 25-OH-24-epi-D₂ (10) when obtained in the free hydroxy forms, are also conveniently hydroxy-protected at the C-3 or C-25, or at both positions, by conventional reactions known in the art. Thus, 25-OH-D₂ may be acylated to yield, for example, the 25-OH-D₂-3-acetate, or the corresponding 3,25-diacetate. The 3-monoacetate, in a like fashion, may be further acylated at C-25 by treatment with a different acylating reagent, or, alternatively, the 3,25-diacetate may be selectively hydrolyzed by a mild base (KOH/MeOH) to give the 25-monoacetate, which if desired can be reacylated with a different acyl group at C-3. Other hydroxy-protecting groups can be introduced by analogous known reactions.

In addition to the hydroxy-protected derivatives, the 5,6-trans-isomers of 25-OH-24-epi-D₂ as well as the 1,25-dihydroxy compounds are compounds of potential utility in medical applications because of their considerable vitamin D-like activity. These 5,6-trans-compounds are prepared from the 5,6-cis-isomers (i.e. 10 or 15) by iodine catalyzed isomerization according to the procedures of Verloop et al. Rec. Trav. Chim. Pays Bas 78, 1004 (1969), and the corresponding 3- and/or 25-hydroxy-protected derivatives are likewise obtained by analogous isomerization of the corresponding 5,6-cis-acylates, or by hydroxy-protection of the 5,6-trans-25-OH-D₂ compounds.

The required side chain fragment, sulfone (21), is itself prepared according to the process shown in Process Scheme II. This synthesis is straight-forward and involves as a first step the reaction of ester (16) in anhydrous tetrahydrofuran (THF) with methyl magnesium bromide to give the diol (17). Diol (17) is dissolved in anhydrous pyridine and reacted with p-toluenesulfonyl chloride to provide the tosylate (18). Tosylate (18) is dissolved in a solution of anhydrous dimethyl formamide and reacted with thiophenol and t-BuOK to yield the sulfide (19). The sulfide (19) in turn is dissolved in dichloromethane and reacted with 3-chloroperoxybenzoic acid to give the hydroxy sulfone compound (20). Pyridinium p-toluenesulfonate is then added to a solution of compound (20) in anhydrous dichloromethane and reacted with dihydropyran to yield the hydroxy protected tetrahydropyranyl sulfone (21a). The corresponding (S)-epimer of sulfone (21) is prepared by the same process, using as starting material the ester corresponding to (16) but having the (S) configuration at carbon-2.

### Non-25-Hydroxylated Compounds (X₂ Is Hydrogen)

The reaction sequence illustrated by Process Scheme III presents another specific embodiment of the overall process, whereas Process Scheme IV illustrates preparation of an appropriate side chain unit for addition to the steroid-22-aldehyde as shown in Scheme III.

Starting materials for the present process are steroidal 22-aldehydes, such as, for example, the PTAD-diene-protected-22-aldehyde (4) shown in Scheme I (where PTAD refers to the phenyltriazoline-3,5-dione-protecting group shown), which in turn can be prepared from ergosterol by the known steps (Scheme I).

The first step of this process comprises the addition of a suitable side chain fragment. Thus, condensation of aldehyde (4) with a sulfonyl-side chain fragment as shown in the Scheme III (sulfone (35), further described below) present in the form of its anion, in an ether or hydrocarbon solvent, provides the hydroxy-sulfone intermediate (22). The anion of the sulfone (35) side chain fragment is generated by treatment of the sulfone with a strong base, such as lithium diethylamide, n-butyl lithium or methyl or ethyl magnesium bromide (or similar Grignard reagent) in an ether or hydrocarbon solvent, and to this solution of sulfone anion the steroid aldehyde (compound 4) as an ether or hydrocarbon solution is then added. The reaction is best effected under an inert atmosphere.

The next step comprises the removal of the hydroxy-and phenylsulfonyl groups in the side chain with formation of the 22(23)-trans-double bond. Thus, treatment of compound (22), in methanol solution saturated with NaHPO₄, with sodium amalgam under an inert atmosphere, gives compound (23) featuring the desired trans-22-double bond in the side chain. If desired, the 22-hydroxy group in compound (22) can also be acylated or sulfonylated (e.g. mesylated) prior to the Na/Hg-reduction step, but this is not generally required.

It is to be noted that, as shown in Process Scheme III, addition of the side chain fragment, sulfone (35), to the aldehyde (4), does not cause epimerization at the asymmetric center of carbon 20, i.e. the stereochemistry at that center is retained, as is required. If desired, retention of stereochemistry at carbon 20 may be checked at this stage of the synthesis by the conversion of intermediates of type (23) back to the original aldehyde starting materials. For example, subjecting compound (23) to ozonolysis with reductive work-up, using fully conventional and standard conditions, yields the corresponding C-22-aldehyde, i.e. the aldehyde of structure (4). Spectroscopic and chromatographic comparison of the aldehyde obtained from ozonolysis with the original starting material confirms retention of C-20 stereochemistry.

The next operation of the process involves conversion of these ring B-protected steroids to the desired 5,7-diene intermediate (24). In the case of the PTAD-diene-protected compound (23), this conversion is accomplished in a single step, namely treatment of (23) with a strong hydride reducing agent (e.g. LiAlH₄) in an ether solvent at reflux temperature gives the diene (24).

Conversion of 5,7-diene (24) to the final vitamin D products (26) or (31) comprises a sequence of several steps. The sequence shown in Process Scheme III involves first the irradiation of an ether or hydrocarbon solution of the 5,7-diene (24) with ultraviolet light to yield the previtamin analog (25) which by warming (50°-90°C) in a suitable solvent (e.g. ethanol, hexane) undergoes isomerization to the vitamin D₂ compound (26).

Thereafter, compound (26) may be converted to the 1α-hydroxyvitamin D₂ compound (31) by the known steps shown in Scheme III. Reference is made to U. S. Patent Nos. 4,260,549 and 4,554,106 for the prior art relevant to these transformations.

The side chain fragment, sulfone (35), as used in Scheme III is specifically the (R) enantiomer. Therefore, compounds (26) or (31) are obtained as the C-24-R-epimers, 24-epi-vitamin D₂ (26) or 1α-hydroxy-24-epi-vitamin D₂ (31), respectively. Thus, compounds (26) or (31) are prepared in epimerically pure form, and C-24-epimer separation as required in the process disclosed in U. S. patent No. 4,448,721, is not necessary. Use of the (S)-epimer of sulfone (35) in the present process yields specifically vitamin D₂, as well as, of course, the respective 1α-hydroxyvitamin D₂ compound.

The 5,7-diene (24) may be used as the free hydroxy compound or as its hydroxy-protected form, where the hydroxy-protecting groups (at C-3) may be acyl, alkylsilyl or alkoxyalkyl groups as previously defined. Thus, the vitamin D₂ product will be obtained as the free hydroxy compound or, if desired, as the C-3-hydroxy-protected derivatives. Synthesis according to Scheme III would provide the vitamin D₂ products as the free hydroxy compounds but analogous conversion of 5,7-diene intermediate (24) as the 3-protected, derivative will yield the corresponding hydroxy-protected derivatives of the vitamin D₂ products.

The individual vitamin D₂ epimers, i.e. vitamin D₂ or 24-epi-D₂ (26) when obtained in the free hydroxy forms, are also conveniently hydroxy-protected at the C-3 position, by conventional reactions known in the art. Thus, vitamin D₂ may be acylated to yield, for example, the vitamin D₂-3-acetate. Other hydroxy-protecting groups can be introduced by analogous known reactions.

In addition to the hydroxy-protected derivatives, the 5,6-trans-isomers of 24-epi-D₂ as well as the 1α-hydroxy compounds are compounds of potential utility in medical applications because of their considerable vitamin D-like activity. These 5,6-trans-compounds are prepared from the 5,6-cis-isomers (i.e. 26 or 31) by iodine catalyzed isomerization according to the procedures of Verloop et al. Rec. Trav. Chim. Pays Bas 78, 1004 (1969), and the corresponding 3-hydroxy-protected derivatives are likewise obtained by analogous isomerization of the corresponding 5,6-cis-acylates, or by hydroxy-protection of the 5,6-trans-D₂ compounds.

The required side chain fragment, sulfone (35), can be prepared according to Perlman et al, supra, or according to the process shown in Process Scheme IV. This synthesis is straightforward and involves as a first step dissolving alcohol (32) in anhydrous pyridine and reacting it with p-toluenesulfonyl chloride to provide the tosylate (33). Tosylate (33) is dissolved in a solution of anhydrous dimethyl formamide and reacted with thiophenol and t-BuOK to yield the sulfide (34). The sulfide (34) in turn is dissolved in dichloromethane and reacted with 3-chloroperoxybenzoic acid to give the sulfone compound (35). The corresponding (S)-epimer of sulfone (35) can also be prepared according to Perlman et al supra, or according to Process Scheme IV, using as starting material the alcohol corresponding to (32) but having the (S) configuration at carbon-2.

### Analog Compounds

Furthermore, the present process also serves as a convenient method for the synthesis of side chain homologated vitamin D₂ analogs where the carbon at any one of the side chain positions designated as 24, 26 and 27 may be homologated, of the formula (40) shown below, or of the corresponding 25-hydroxy-analogs and/or 1α-hydroxy-analogs, 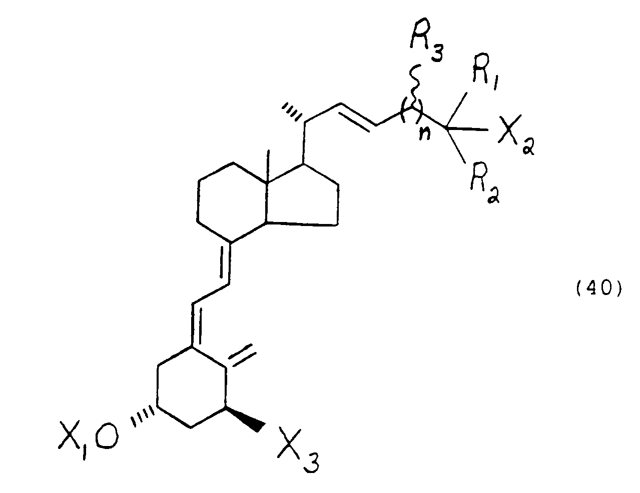
where n is an integer having a value of from 1 to 5, X₁ is selected from hydrogen and a hydroxy-protecting group, X₂ is selected from hydrogen, hydroxy and protected hydroxy, X₃ is selected from hydrogen, hydroxy, and protected hydroxy, R₃ is alkyl, hydroxy, protected hydroxy, hydrogen or fluorine, and where R₁ and R₂, which may be the same or different, is an alkyl group or an aryl group and where the configuration about carbon 24 has either the (R)- or the (S)-stereochemical orientation. These compounds are prepared by condensing compound (4) with the appropriate alkyl or aryl side chain fragment as shown by the following formulae, 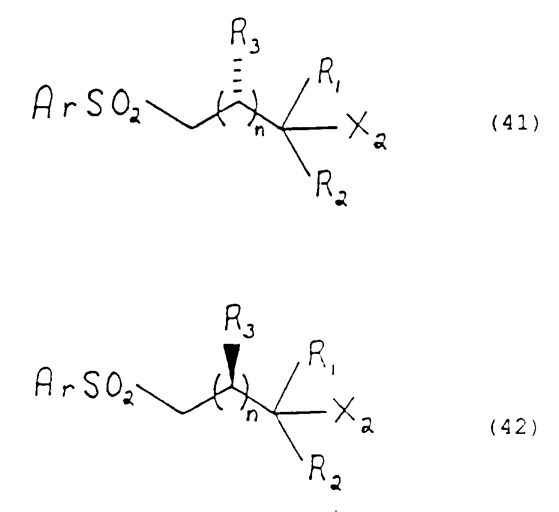
where X₂, R₁, R₂, R₃ and n are as defined above.

The use of compounds where (41) and (42) as side chain units in the synthetic process depicted as in Schemes I and III, then provides the 25-OH-D₂- or 25-OH-24-epi-D₂-homologs of general structure (40) where R₁, R₂ and R₃ represent alkyl or aryl residues or other substitutes as defined above. The products of general structure (40) can there be 1α-hydroxylated according to known methods (See U. S. Patent Nos. 4,260,549 and 4,554,106) so as to obtain the corresponding 1α-hydroxylated vitamin D homologs.

Since the compounds where R₁, R₂ or R₃ represent higher homologs of methyl, are generally more lipophilic, the alkyl- or aryl-analogues represented by structure (40) above or their 5,6-trans-isomers, are expected to have utility in applications where a greater degree of lipophilicity is desired.

The present invention is further described by means of the following illustration. In this illustration, numerals designating specific products, e.g. compounds 1, 2, 3 etc. refer to the structures so numbered in Process Schemes I or II.

### EXAMPLE I

### Ergosterol Method

To a solution of 50g (0.13mol) of ergosterol 1 in 300ml of anhydrous pyridine was added 33.3ml (0.35mol) of acetic anhydride. The mixture was stirred at room temperature overnight and 600ml of water was added. The precipitate was filtered off, washed several times with 200ml of water and recrystallized from ethanol to obtain 2, 42.0g (76%). [yellowish crystal]
To a solution of 33g (0.075mol) of 2 in 500ml of chloroform was added 13.2g (0.075mol) of 4-phenyl-1,2, 4-triazoline-3,5-dione. The solution was stirred at room temperature for 30 min and 5ml of pyridine was added. The solution was cooled at -78°C and treated with an ozone-oxygen mixture for 30 min (TLC control) and thoroughly purged with nitrogen. 50ml of dimethyl sulfide was added and the mixture was washed with 300ml of water, 200ml of 2N HCl (twice) and 300ml of water. The organic layer was separated and each washing was extracted with 400ml and 200ml of chloroform. The combined extract was dried over Na₂SO₄ and concentrated in vacuo. The residue was purfied on a silica gel column (5.06X63cm, 550g of 150∼ 425»m silica gel) using a mixture of ethyl acetate and hexane as eluant. 20.5g (50%) of 4 was eluted with 30% ethyl acetate in hexane. To increase yield, the recovered 3 (eluted with 15% ethyl acetate in hexane) was treated with an ozone-oxygen mixture as above.

To a stirred solution of 12.1g (37.1mmol) of sulfone 21, 5.10ml (36.4mmol) of diisopropyl amine and 100ml of anhydrous tetrahydrofuran (containing 1,10-phenanthroline as indicator) under nitrogen atmosphere at -78°C was added 22.7ml (36.3mml) of n-BuLi (1.6M in hexane). The solution was stirred under nitrogen at -78°C for 30min, then 10.0g (18.3mmol) of 4 in 40ml of anhydrous tetrahydrofuran was added. The mixture was stirred at -78°C for 1hr, decomposed by the addition of 100ml of saturated NH₄Cl solution, warmed to 0°C and extracted three times with 100ml of ethyl acetate. Each extract was washed with 100ml of saturated NaCl solution, dried over Na₂SO₄ and concentrated in vacuo. The residue was purified on a silica gel column (3.2X60cm, 150g of 75∼ 150»m silica gel). The unreacted sulfone 21 was eluted with benzene and 14.7g (92%) of 5 was eluted with ethyl acetate.

A mixture of 14.7g (16.9mmol) of hydroxysulfone 5, 110g of 5% sodium amalgam and 400ml of methanol saturated with Na₂HPO₄ was stirred under nitrogen atmosphere at 5°C for 20 hrs. The reaction solution was decanted and concentrated in vacuo. The residue was dissolved in 200ml of ethyl acetate and washed with 400ml and 200ml of water. The ethyl acetate extract was separated and each of washing was extracted twice with 200ml of ethyl acetate. The combined extract was dried over Na₂SO₄ and concentrated in vacuo. 10.5g (91%) of 6 was obtained.

To a solution of 10.5g (15.4mmol) of 6 in 400ml of tetrahydrofuran was added 11.5g (303.0mmol) of LiAlH₄. The mixture was heated under reflux and nitrogen atmosphere for 3 hrs, cooled with ice water and decomposed by the dropwise addition of 40ml of ethyl acetate and 60ml of water. Then, the mixture was filtered and the filtrate was concentrated in vacuo. The residue was dissolved in 200ml of ethyl acetate and washed twice with 200ml of saturated NaCl solution. The ethyl acetate extract was separated and each washing was extracted with 200ml of ethyl acetate. The combined extract was dried over Na₂SO₄ and concentrated in vacuo. Then, the residue was purified on a silica gel column (3.2X25cm, 80g of 75 150»m silica gel) and 4.8g (63%) of 7 was eluted with 5% ether in benzene as eluant [yellow foam].

To a solution of 4.4g (8.9mmol) of 7 in 200ml of methanol and 130ml of dichloromethane was added 2.0g (7.9mmol) of pyridinium p-toluenesulfonate. The mixture was stirred at room temperature overnight, dissolved in 300ml of saturated NaCl solution and extracted three times with 400ml of dichloromethane. Each extract was washed with 400ml of saturated NaCl solution, combined, dried over Na₂SO₄ and concentrated in vacuo. The residue was recrystallized from ethanol to obtain 2.5g (68%) of 8 [white crystal]. To increase yield, the mother liquor was concentrated in vacuo, purified with chromatography and recrystallized from ethanol.

1.50g (3.6mmol) of 8 was dispersed in 500ml of a mixture of ether and benzene (4:1) and irradiated with stirring under nitrogen in a water-cooled quartz immersion well equipped with an ozone free filter using Eikosha's high-pressure UV lamp for 25min. The reaction was monitored by HPLC using Lichrosorb Si 60 (5 »m) column and 3% 2-propanol in hexane at 265nm.

The solution was concentrated in vacuo, redissolved in 100ml of ethanol and heated under reflux and nitrogen for 3 hrs. Then, the solution was concentrated in vacuo and the residue was purified on a silica gel column (3.2X50cm, 170g of 75∼ 150 »m of silica gel) using a mixture of ethyl acetate in hexane. 074g (50%) of 10 was eluted with 20% ethyl acetate in hexane. [white foam]
To a solution of 1.50g (3.6mmol) of 10 in 15ml of anhydrous pyridine was added 1.50g (7.9mmol) of tosyl chloride. The mixture was stirred under nitrogen at 5°C for 20 hrs. Then, the solution was poured into 200ml of cold saturated NaHCO₃ solution. The mixture was allowed to stand for 30 min and extracted three times with 150ml of a mixture of ether and dichloromethane (4:1). Each extract was washed with 150ml of saturated NaCl solution, 150ml of cold diluted HCl solution for twice, 150ml of saturated NaCl solution, 150ml of saturated NaHCO₃ solution and 150ml of saturated NaCl solution. The combined extract was dried over Na₂SO₄ and concentrated in vacuo. 1.90g (92%) of 11 was obtained and converted to 12 without further purification. [white foam]
4.40g of anhydrous KHCO₃ was dissolved in 200ml of anhydrous methanol under nitrogen at 50°C. To this solution was added dropwise a solution of 1.90g (3.4mmol) of 11 in 30ml of anhydrous dichloromethane. The mixture was stirred under nitrogen at 50°C for 21 hrs. Then, the solution was concentrated in vacuo, the residue was dissolved in 200ml of a mixture of ether and dichloromethane (4:1) and washed twice with 100ml of water. The organic extract was separated and each washing was extracted twice with 100ml of the same mixture of ether and dichloromethane. The combined extract was dried over Na₂SO₄ and concentrated in vacuo to obtain 1.50g (105%) of 12 which was hydroxylated without further purification. [yellow oil]
2.7ml (8.1mmol) of tert-butyl hydroperoxide (3.0M in 2,2-4-trimethylpentane) was added to a suspension of 220mg (2.0mmol) of selenium dioxide in 75ml of anhydrous dichloromethane. The mixture was stirred at room temperature under nitrogen for 30min. 0.3ml of anhydrous pyridine was added followed by a solution of 1.50g (3.5mmol) of 12 in 30ml of anhydrous dichloromethane. The mixture was stirred under nitrogen at room temperature for 30min and heated under reflux for 10min. Then, 50ml of 10% NaOH solution was added and the mixture was extracted with 200, 100 and 100ml of ether. Each extract was washed with 50ml of 10% NaOH solution and 50ml of saturated NaCl solution. The combined extract was dried over Na₂SO₄ and concentrated in vacuo. The residue was purified on a silica gel column (3.2cmX15cm, 50g of 75∼ 150 »m silica gel) using a mixture of ethyl acetate in hexane as eluant. 581mg (37%) of 13 was eluted with 30% ethyl acetate in hexane. [yellow oil]
581mg (1.3mmol) of 13 was dissolved in 5ml of acetic acid and heated at 50°C under nitrogen for 1 hr. Then, the solution was poured over ice and neutralized with 100ml of saturated NaHCO₃ solution. The mixture was extracted three times with 150ml of a mixture of ether and dichlormethane (4:1) Each extract was washed with 100ml of saturated NaHCO₃ solution and 100ml of saturated NaCl solution. The combined extract was dried over Na₂SO₄ and concentrated in vacuo. Then, to a solution of the residue in 10ml of ethyl acetate was added 120mg of maleic anhydride and the mixture was allowed to stand under nitrogen at room temperature for 2 hrs. Then, the solution was concentrated in vacuo, the residue was redissolved in 50ml of ether. 50ml of 0.1N KOH in methanol was added, the solution was stirred at room temperature for 1.5 hrs. and concentrated in vacuo. The residue was dissolved in 100ml of a mixture of ether and dichloromethane (4:1) and washed with 50ml of 10% NaOH solution for twice and 50ml of saturated NaCl solution. The organic extract was separated and each of washing was extracted twice with 100ml of the same mixture of ether and dichloromethane. The combined extract was dried over Na₂SO₄ and concentrated in vacuo. The residue was purified on a silica gel column (2.3X8.0cm, 10g of 45∼75 »m silica gel) using a mixture of ethyl acetate in hexane as eluant. 310 mg of 15 was eluted with 30% ethyl acetate in hexane, combined with another 337mg of 15 and recrystallized from methyl formate.

### EXAMPLE 2

### Intermediates for Side Chain

20g (0.169mol) of methyl (R)-(-)-3-hydroxy-2-methyl propionate 16 was dissolved in 60ml of anhydrous tetrahydrofuran and added under nitrogen atmosphere and ice cooling to a stirred solution of 245ml (0.735mol) of methylmagnesium bromide (3.0M solution in ether). At the end of the addition 100ml of anhydrous tetrahydrofuran was added to facilitate stirring. The mixture was stirred at room temperature for 2 hrs, decomposed by the careful addition of 150ml of 5N HCl with ice cooling and extracted three times with 200ml of ether. Each extract was washed with 150ml of saturated NaCl solution, combined and dried over Na₂SO₄. Evaporation afforded 16.4g (82%) of 17 as yellow oil.

A mixture of 16.4g (0.139mol) of 17, 26.5g (0.139mol) of tosyl chloride and 30ml of pyridine was stirred at 4°C overnight. Then, the reaction mixture was dissolved in 300ml of ether and washed with 200ml of water, 200ml of diluted HCl, 200ml of water and 200ml of saturated NaHCO₃ solution. The ether extract was separated and each washing was extracted twice with 200ml of ether. The combined extract was dried over Na₂SO₄ and concentrated in vacuo. The residue was purified on a silica gel column (5.5X20cm, 200g of 150 425m silica gel) and 32.1g (85%) of tosylate (18) was eluted with 10∼20% ethyl acetate in hexane. [red oil]
To a stirred solution of 14.4g (0.131mol) of thiophenol in 70ml of anhydrous dimethyl formamide 14.4g (0.131mol) of t-BuOK was added followed by 32.1g (0.118mol) of 18 in 90ml anhydrous dimethyl formamide. The mixture was stirred overnight, dissolved in 300ml of ice water and extracted with 300,200 and 200ml of ethyl acetate. Each extract was washed with 200ml saturated NaHCO₃ solution and water, combined, dried over Na₂SO₄ and concentrated in vacuo. 28.0g (113%, containing dimethyl formamide) of 19 was obtained and was oxidized without further purification. [red oil]
28.0 (0.118mol) of 19 was dissolved in 400ml of dichloromethane and cooled with ice water. To this solution was added 51.7g (0.300mol) of m-chloroperbenzoic acid slowly, the mixture was stirred at room temperature for 2 hrs. and filtered. The filtrate was washed with 300ml of saturated NaHCO₃ solution for twice, 300ml of saturated Na₂SO₃ solution for twice and 300ml of saturated NaHCO₃ solution. The organic phase was separated and each washing was extracted twice with 300ml of dichloromethane. The combined extract was dried over Na₂SO₄, concentrated in vacuo and recrystallized from a mixture of ethyl acetate in hexane to obtain 20 25.2g (88%). [white crystals]
To a stirred solution of 20g (0.083mol) of 20 in 50ml of dichloromethane was added 20ml (0.221mol) of freshly distilled 2,3-dihydropyran followed by 0.8g of pyridinium p-toluenesulfonate. The mixture was stirred at room temperature for 2 hrs. and washed twice with saturated NaCl solution. The organic phase was separated and each of washing was extracted twice with 50ml of dichloromethane. The combined extract was dried over Na₂SO₄ and concentrated in vacuo. The residue was purified on a silica gel column (3.2X45cm, 150g of 75 ∼ 150 »m silica gel) and 26.0g (96%) of 21a was eluted with benzene as eluant. [colorless oil]

### EXAMPLE 3

To a solution of ergosterol 1 in anhydrous pyridine is added acetic anhydride. The mixture is stirred at room temperature overnight and water is added. The precipitate is filtered off, washed several times with water and recrystallized from ethanol to obtain 2.

To a solution of the precipitate 2 in chloroform is added 4-phenyl-1, 2, 4-triazoline-3,5-dione. The solution is stirred at room temperature and pyridine is added. The solution is cooled and treated with an ozone-oxygen mixture (TLC control) and thoroughly purged with nitrogen. Dimethyl sulfide is added and the mixture is washed with water, 2N HCl and then water again. The organic layer is separated and each washing is extracted with chloroform. The combined extract is dried over Na₂SO₄ and concentrated in vacuo. The residue is purified by silica gel chromatography to obtain compound 4.

To a stirred solution of sulfone 35, diisopropyl amine and anhydrous tetrahydrofuran (containing 1,10-phenanthroline as indicator) under nitrogen atmosphere is added n-BuLi (1.6M in hexane). The solution is stirred under nitrogen, then, compound 4 in anhydrous tetrahydrofuran is added. The mixture is stirred, decomposed by the addition of saturated NH₄Cl solution, warmed and extracted three times with ethyl acetate. Each extract is washed with saturated NaCl solution, dried over Na₂SO₄ and concentrated in vacuo. The residue is purified on a silica gel column to afford compound 22.

A mixture of hydroxysulfones 22, 5% sodium amalgum and methanol saturated with Na₂HPO₄ is stirred under nitrogen atmosphere. The reaction solution is decanted and concentrated in vacuo. The residue is dissolved in ethyl acetate and washed with water. The ethyl acetate extract is separated and each washing is extracted twice with ethyl acetate. The combined extract is dried over Na₂SO₄ and concentrated in vacuo to obtain 23.

To a solution of compound 23 in tetrahydrofuran is added LiAlH₄. The mixture is heated under reflux and nitrogen atmosphere, cooled with ice water and decomposed by the dropwise addition of ethyl acetate and water. Then, the mixture is filtered and the filtrate is concentrated in vacuo. The residue is dissolved in ethyl acetate and washed twice with saturated NaCl solution. The ethyl acetate extract is separated and each washing is extracted with ethyl acetate. The combined extract is dried over Na₂SO₄ and concentrated in vacuo. Then, the residue is purified on a silica gel column to provide compound 24.

Compound 24 is dispersed in a mixture of ether and benzene (4:1) and irradiated with stirring under nitrogen in a water-cooled quartz immersion well equipped with an ozone free filter using a high-pressure UV lamp. The reaction may be monitored by HPLC.

The solution is concentrated in vacuo, redissolved in ethanol and heated under reflux and nitrogen. Then, the solution is concentrated in vacuo and the residue is purified on a silica gel column to obtain compound 26.

To a solution of compound 26 in anhydrous pyridine is added tosyl chloride. The mixture is stirred under nitrogen. Then, the solution is poured into a cold saturated NaHCO₃ solution. The mixture is allowed to stand for 30min and extracted three times with a mixture of ether and dichloromethane (4:1). Each extract is washed with saturated NaCl solution, cold diluted HCl solution twice, saturated NaCl solution, saturated NaHCO₃ solution and saturated NaCl solution. The combined extract is dried over Na₂SO₄ and concentrated in vacuo. Compound 27 is obtained and converted to 28 without further purification.

Anhydrous KHCO₃ is dissolved in anhydrous methanol under nitrogen. To this solution is added dropwise a solution of compound 27 in anhydrous dichloromethane. The mixture is stirred under nitrogen. Then, the solution is concentrated in vacuo, the residue is dissolved in a mixture of ether and dichloromethane (4:1) and washed twice with water. The organic extract is separated and each washing is extracted twice with the same mixture of ether and dichloromethane. The combined extract is dried over Na₂SO₄ and concentrated in vacuo to obtain Compound 28 which is hydroxylated without further purification.

Tert-butyl hydroperoxide (3.0M in 2,2-4-trimethylpentane) is added to a suspension of selenium dioxide in anhydrous dichloromethane. The mixture is stirred at room temperature under nitrogen. Anhydrous pyridine is added followed by a solution of compound 28 in anhydrous dichloromethane. The mixture is stirred under nitrogen at room temperature and heated under reflux. Then, a 10% NaOH solution is added and the mixture is extracted with ether. Each extract is washed with a 10% NaOH solution and a saturated NaCl solution. The combined extract is dried over Na₂SO₄ and concentrated in vacuo. The residue is purified on a silica gel column to obtain compound 29.

Compound 29 is dissolved in acetic acid and heated under nitrogen. Then, the solution is poured over ice and neutralized with saturated NaHCO₃ solution. The mixture is extracted three times with a mixture of ether and dichloromethane (4:1). Each extract is washed with a saturated NaHCO₃ solution and a saturated NaCl solution. The combined extract is dried over Na₂SO₄ and concentrated in vacuo. Then, to a solution of the residue in ethyl acetate is added maleic anhydride and the mixture is allowed to stand under nitrogen at room temperature. Then, the solution is concentrated in vacuo, the residue is redissolved in ether. KOH in methanol is added, the solution is stirred at room temperature and concentrated in vacuo. The residue is dissolved in a mixture of ether and dichloromethane (4:1) and washed with 10% NaOH solution twice and a saturated NaCl solution. The organic extract is separated and each washing is extracted twice with the same mixture of ether and dichloromethane. The combined extract is dried over Na₂SO₄ and concentrated in vacuo. The residue is purified on a silica gel column using a mixture of ethyl acetate in hexane as eluant to obtain compound 31.

### EXAMPLE 4

### Intermediates for Side Chain

A mixture of compound 32, tosyl chloride and pyridine is stirred overnight. Then, the reaction mixture is dissolved in ether and washed with water, diluted HCl, water and saturated NaHCO₃ solution. The ether extract is separated and each washing is extracted twice with ether. The combined extract is dried over Na₂SO₄ and concentrated in vacuo. The residue is purified on a silica gel column to give tosylate (33).

To a stirred solution of thiophenol in anhydrous dimethyl formamide t-BuOK is added followed by compound 33 in anhydrous dimethyl formamide. The mixture is stirred overnight, dissolved in ice water and extracted with ethyl acetate. Each extract is washed with a saturated NaHCO₃ solution and water, combined, dried over Na₂SO₄ and concentrated in vacuo. Compound 34 is obtained and oxidized without further purification.

Compound 34 is dissolved in dichloromethane and cooled with ice water. To this solution is added m-chloroperbenzoic acid slowly, the mixture is stirred at room temperature for 2 hrs. and filtered. The filtrate is washed with a saturated NaHCO₃ solution twice, a saturated Na₂SO₃ solution twice and a saturated NaHCO₃ solution. The organic phase is separated and each washing is extracted twice with dichloromethane. The combined extract is dried over Na₂SO₄, concentrated in vacuo and recrystallized from a mixture of ethyl acetate in hexane to form compound 35.

### EXAMPLE 5

### Alternative Synthesis

As an alternative to the above-described synthesis, the compounds of the present invention may be prepared in accordance with the procedures set forth in U. S. Patent No. 4,847,012. The following illustrates the preparation of 24-epi-26,27-dihomo 1α,25-dihydroxyvitamin D₂. This compound is distinguished from the previously known 24-epi-1α,25-dihydroxyvitamin D₂ by homologation at the 26,27 positions. As is readily apparent to those skilled in this art, the following described synthesis may be readily adapted to making the other homologated compounds disclosed herein by selecting the appropriate side chain unit and/or vitamin D nucleus for the condensation step.

The synthesis of 24-epi-26,27-dihomo-1α,25-dihydroxyvitamin D₂ requires the construction of an appropriate side chain unit having the desired (S) stereochemistry at the carbon center that is to become carbon-(24 R) in the target compound, and the condensation of the side chain unit with a suitable 1α-hydroxylated vitamin D nucleus so as to generate the desired final product.

Referring first to Scheme 5, the synthesis of the optically active side chain unit comprised the conversion of the commercially available (R)-(-)-3-hydroxy-2-methylpropionate 41 with ethylmagnesium bromide to the diol 42. This was converted to tosylate 43, then treated with potassium thiophenoxide in alkaline DMF to give sulfide 44. This in turn was oxidized with 3-chloroperbenzoic acid to afford sulfone 45. The very hindered hydroxy of sulfone 45 could not be protected by standard methodology. However, it was successfully protected by the use of triethylsilyltriflate in triethylamine to give the protected sulfone 46.

For the preparation of the desired 24-epi-26,27-dihomo-1α,25-dihydroxyvitamin D₂, one is referred to Scheme 6. In Scheme 6, (4S)-3-ethyl-2-methyl-5-phenylsulfonyl-3-triethylsilyloxypentane 46, as obtained by the above procedure, was reacted with the known 1α-hydroxyvitamin D-22 aldehyde derivative 47, using the general procedures of Kutner et al., J. Org. Chem., 53, 3450 (1988). This condensation yields the side chain adduct represented by structure 48, which is then reduced with a metal amalgam to provide the hydroxy protected (24R)-26,27-dihomo-1α,25-dihydroxyvitamin D₂ 49. Upon removal of the hydroxy protecting groups according to standard procedures the desired 24-epi-26,27-dihomo-1α,25-dihydroxyvitamin D₂ 50 is obtained.

### (4S)-3-Ethyl-4-methyl-5-phenylsulfonyl-3-(triethylsilyloxy)-pentane (46)

A solution of 3.3 g (28 mmol) methyl (R)-(-)-3-hydroxy-2-methylpropionate 41 in 5 mL of anhydrous THF was cooled to 0°C and added dropwise with vigorous stirring, over 15 min, under argon, to a solution of ethylmagnesium bromide (55 mL of 2 M in THF) at -10°C. The reaction mixture was stirred 2 h at room temperature, then quenched with 15 mL of 1:1 diluted HC1. The water phase was extracted with ether, dried over anh. MgSO₄, filtered and evaporated to give 3.0g (74%) of the crude diol 42.

The crude diol 42 (3 g)(20.5 mmol) was dissolved in 8 mL of anhydrous pyridine and 4.7 g (24.7 mmol) of p-toluenesulfonyl chloride was added with stirring at 0°C. The mixture was kept for 16 h at 4°C and quenched with ice water. The water phase was extracted with ether and the ether phase was washed with ice cold 1 N. HC1, saturated CuSO₄ solution, water, NaHCO₃ solution, brine and dried over anh. MgSO₄, filtered and evaporated. The crude oil was purified by silica gel column chromatography with ethyl acetate - hexane mixtures to give 4.4 g (73%) tosylate 43 as a colorless oil.

To a stirred solution of 1.62 g (14.7 mmol) of thiophenol and 1.65 g (14.7 mmol) of potassium tert-butoxide in 5 mL of anhydrous dimethylformamide was added 3.94 g (13.1 mmole) of the tosylate 43 in 2 mL of anhydrous dimethylformamide. The reaction mixture was stirred at room temperature overnight, poured on water and extracted with ether. The organic layer was washed with aqueous sodium carbonate and water and dried (MgSO₄). Solvents were removed in vacuo and the crude oil was purified by silica gel chromatography with hexane-ethyl acetate to give sulfide 44 (2.5 g)(85%) as a colorless oil.

Sulfide 44 (2.4 g. 10 mmol) was then dissolved in 36 mL of dry dichloromethane and 3.0 g (17.4 mmol) 3-chloroperbenzoic acid was added in portions with occasional cooling. The mixture was stirred for 2 h and quenched with 10% sodium bicarbonate. The combined organic extracts were washed with aqueous sodium sulfite, and brine and dried over anh. MgSO₄. The solvent was removed in vacuo and the crude oil was purified by silica gel flash chromatography using hexane ethyl acetate to afford sulfone 45 1.98 g (67%) as a colorless liquid. [α]²²D : +9.2 (c=5.2, CHCl₃) Anal.Calcd for C₁₄H₂₂O₃S: C, 62.19; H, 8.20; S, 11.86. Found: C, 62.26; H, 8.14; S, 11.85, ¹H NMR (500 MHz, CDCl₃) δ 1.10 (3H,m, 4-CH₃) 2.30 (1H,m,4-CH), 2.84 (1H,m, 5-CH) 3.52 (1H,m, 5-CH), 7.57, 7.66 and 7.92 (5H, m, Ar-H). Mass spectrum m/z (rel. intensity) 271 (M⁺ + 1)(10), 265 (8), 253 (100), 241.

0.87 g (3.2 mmol) dry sulfone 45 was dissolved in 5 mL of anhydrous dichloromethane, cooled to 0°C and 5 mL of anhydrous triethylamine added followed by 3 mL (13 mmol) of triethylsilyl triflate under argon and 0°C. The mixture was stirred at room temperature for 1.5 h, ether added and the ether phase washed with ice cold water, brine and dried (MgSO₄) and evaporated. The residue was purified by flash chromatography with 10% ethyl acetate in hexane to give 1.45 g (99%) of the triethylsilylated sulfone 46. ¹H NMR (500 MHz, CDCl₃) δ 1.09 (3H, m, 4-CH₃), 2.28 (1H, m, 4-CH), 2.85 (1H, m, 5-CH), 3.46 (1H, m, 5-CH). Mass spectrum m/z (relative intensive) 383 (38), 369 (100), 355 (99), 325 (35) 77, 59.

### 24-Epi-26,27-Dihomo-1α,25-dihydroxyvitamin D₂ (50).

To a stirred solution of 24 mg (42 »mol) triethysilyloxysulfone 46 in 300 »L anhydrous tetrahydrofuran containing 1,10-phenanthroline as an indicator was added under argon at -78°C 8 »L (60 »mol) diisopropylamine followed by 32 »L (51 »mol) of a solution of 1.5 M n.BuLi in hexane. The solution was stirred at -78°C for 30 min (orange-brown color) and 5 mg (7.7 »mol) of protected aldehyde 47 in 300 »L anhydrous tetrahydrofuran was added and the mixture stirred under argon at -78°C for 1 h. The mixture was quenched with cold saturated NH₄Cl solution, warmed to 0°C and extracted with ethyl acetate, and the organic phase washed with saturated NaCl solution. The organic phase was dried over anhydrous MgSO₄, filtered and evaporated. The residue was purified by preparative HPLC (Zorbax Sil 9.4 x 25 cm column) using 10% ethylacetate in hexane 108 mg of recovered aldehyde 47 and 6 mg of crude product 48.

A saturated solution of Na₂HPO₄ in methanol (1 mL) was added to a stirred solution of 1.2 mg hydroxysulfone 48 in 10 mL of anhydrous tetrahydrofuran, followed by 160 mg of powdered anhydrous Na₂HPO₄. The mixture was cooled to 0°C and fresh 5% sodium amalgam (cca 400 mg) was added. The mixture was stirred at 5°C for 20 h. A mixture of 1:1 hexane ethyl acetate (5 mL) was then added and the organic layer decanted. The solid material was 3 x more extracted with ethyl acetate-hexane. The combined organic phase was washed with saturated NaCl solution and filtered through a SepPak cartridge and evaporated. Final purification by HPLC (Zorbax Sil 9.4 x 25 cm column) (10% ethyl acetate in hexane) gave 0.650 mg (66%) of the protected vitamin D₂ analogue 49.

49 was dissolved in 0.5 mL of anhydrous tetrahydrofuran followed by the addition of 0.5 mL of tetrabutylammonium fluoride in tetrahydrofuran. The mixture was stirred under argon at 55°C for 1 h, cooled and 5 mL of ether added. The organic phase was washed with 10% solution of NaHCO₃, brine and dried over anhydrous MgSO₄, filtered and evaporated. The residue was dissolved in 20% 2-propanol in hexane and passed through a silica gel SepPak column. Preparative HPLC (Zorbax Sil 9.4 x 25 cm column) in 20% 2-propanol in hexane gave 126 »g (35%) 24-epi-26,27-dihomo-1α,25-dihydroxyvitamin D₂ 50.

24-epi-26,27-dihomo-1,25-dihydroxyvitamin D₂ exhibited the following spectral properties: UV (EtOH) λₘₐₓ 264nm, λₘᵢₙ 228nm; MS m/z (relative intensity) 456 (M⁺,6), 438 (10), 420 (8), 402 (4), 352 (10), 269 (6), 251 (8), 135 (28), 134 (33), 87 (100), 57 (35); ¹H NMR (CDCl₃) δ 0.55 (3H, S 18-CH₃), 0.97 (3H, d, J=6.9 Hz, 28-CH₃), 1.01 (3H,d, J=6.6 Hz, 21-CH₃), 4.23 (1H,m, 3-H), 4.43 (1H,m, 1-H), 4.99 (1H, br s, 19Z-H), 5.30 (2H, m, 22-H and 23-H), 5.31 (1H, br,s. 19E-H), 6.01 (1H, d, J=11.25 Hz, 7-H), 6.37 (1H, d, J=11.3 Hz, 6-H). Exact mass calcd for C₃₀H₄₈O₃ : 456.3603. Found 456.3603.

### Biological Activity of 26,27-Dihomo-1α-Hydroxyvitamin D₂; 26,27-Dihomo-1α-Hydroxy-24-epi-Vitamin D₂; and 26,27-Dihomo-1,25-Dihydroxy-24-Epi-Vitamin D₂

The new analogs were tested in the vitamin D-deficient rat. These tests indicate that 26,27-dihomologation of 1α-hydroxyvitamin D₂, 1α-hydroxy-24-epi-vitamin D₂ and 1,25-dihydroxy-24-epi-vitamin D₂ had a biological activity spectrum that is very different from previously known 26,27-dihomologated 1,25-dihydroxyvitamin D₃ or 22-dehydro-1,25-dihydroxyvitamin D₃ compounds. In Tables 1-4 below, representative assay results are given. These include tests of intestinal calcium transport activity (S/M ratios), and of bone mineralization as reflected by serum calcium levels. These assays were conducted according to standard procedures (see e.g. U.S. Patent 4,586,716). Table 5 illustrates the cell differentiation activity of 26,27-dihomo-24-epi-1α-hydroxyvitamin D₂ and 26,27-dihomo-1α-hydroxyvitamin D₂ as compared with 26,27-dihomo-24-epi-1,25-dihydroxyvitamin D₃. The NBT (nitroblue tetrazolium reduction) and phago (phagocytosis assay) differentiation data of HL-60 cells were obtained from tessts conducted according to standard procedures (see e.g. U. S. Patent No. 4,973,584).

The data in Table 1 shows that 26,27-dihomo-24-epi-1α,25-dihydroxyvitamin D₂ exhibited high activity in stimulating intestinal calcium transport as well as in mobilizing calcium from bone. The activity of this D₂ analog is shown to be about the same as for 1,25-dihydroxyvitamin D₃.

The data in Tables 2-4 show that the new analog, 26,27-dihomo-1α-hydroxyvitamin D₂ and 26,27-dihomo-24-epi-1α-hydroxyvitamin D₂ exhibited little or no activity in mobilizing calcium from bone or stimulating intestinal calcium transport. However, as seen in Table 4, the 26,27-dihomo-24-epi-1α-hydroxyvitamin D₂ compound decreases bone ash below control values, suggesting that it antagonizes remaining endogenous vitamin D. Similarly, this compound diminishes serum calcium concentration below vitamin D-deficient control values, providing additional evidence that this compound serves as a vitamin D antagonist. These results suggest that this compound could be of considerable importance as an anti-hypercalcemia drug, and can be used in such conditions of neoplastic hypercalcemia, hyperparathyroidism, general hypercalcemia, and vitamin D intoxication hypercalcemia.

The data in Table 5 shows that 26,27-dihomo-1α-hydroxyvitamin D₂ has relatively high differentiation activity, i.e. approximately equal to 26,27-dihomo-24-epi-1,25-dihydroxyvitamin D₃. In contrast, 26,27-dihomo-24-epi-1α-hydroxyvitamin D₂ has little, if any, differentiation activity.

**TABLE 1**

| Response of Vitamin D-Deficient Rats to a Single Dose of 26,27-Dihomo-24-Epi-1α,25-(OH)₂D₂ | | | |
|---|---|---|---|
| Group | Amount | Ca Transport (Serosal/Mucosal) | Serum Calcium (mg%) |
| -D (Control | 0 | 2.6 ± 0.21 | 4.8 ± 0.11 |
| 1,25-(OH)₂D₃ | 50 ng | 3.8 ± 0.44 | 5.5 ± 0.12 |
| | 125 ng | 4.5 ± 0.33 | 5.9 ± 0.15 |
| 26,27-dhomo- | 50 ng | 3.8 ± 0.25 | 5.2 ± 0.11 |
| 24-Epi-1α,25-(OH)₂D₂ | 125 ng | 4.3 ± 0.21 | 6.0 ± 0.09 |

Each group consisted of 6 rats. Results are mean ± SEM.

Animals were maintained on 0.2% Ca, .3% phosphorus for 3-4 weeks prior to experimentation. Vitamin D compounds were administered in 50 »l of ethanol intrajugularly, and 19 hours later, the rats were killed for determination of serum calcium and intestinal calcium transport.

**TABLE 2**

| Intestinal Calcium Transport and Bone Calcium (Serum Calcium) Mobilization Activities of 26,27-Dihomo-1α-Hydroxyvitamin D₂ and 26,27-Dihomo-24-Epi-1α-Hydroxyvitamin D₂ | | | |
|---|---|---|---|
| Compound | Dose (pmoles) | Intestinal Ca Transport (S/M Ratio) | Serum Calcium (Bone Ca Mobil.) (mg%) |
| -D (Control) | 0 | 2.5 ± 3.5 | 3.7 ± 0.20 |
| 1α-OH-D₂ | 325 | 5.4 ± 0.37 | 5.3 ± 0.15 |
| 24-Epi-1α-OH-D₂ | 325 | 4.3 ± 0.42 | 3.9 ± 0.39 |
| | 650 | 4.4 ± 0.70 | 4.1 ± 0.23 |
| -D (Control) | 0 | 3.4 ± 0.2 | 4.1 ± 0.1 |
| 26,27-Dihomo-1α-OH-D₂ | 100 | 3.6 ± 0.2^{b} | 4.6 ± 0.2^{b} |
| | 325 | 3.6 ± 0.3^{b} | 4.1 ± 0.2^{b} |
| 26,27-Dihomo-1α-OH-24-epi-D₂ | 100 | 4.3 ± 0.3^{a} | 4.2 ± 0.1^{b} |
| | 325 | 4.4 ± 0.3^{a} | 4.2 ± 0.1^{b} |
| 1α-OH-D₂ | 100 | 5.7 ± 0.3^{a} | 4.9 ± 0.1^{a} |
| | 325 | 5.5 ± 0.2^{a} | 5.1 ± 0.07^{a} |

| | | | |
|---|---|---|---|
| ^{a}Significant difference compared to respective control groups; p < 0.001. | | | |
| ^{b}No significant difference compared to control. | | | |

Rats were fed a 0.02% Ca, 0.3% P diet for 3 weeks and then given the indicated dose (100 pmol) intravenously or (325 pmol) intraperitoneally. The measurements were made 12.5 hours after the dose. There were 6 rats per group.

**TABLE 3**

| Calcium Transport and Bone Calcium Mobilization of Rats Chronically Dosed With 26,27-Dihomo-24-Epi-1α-OH-D₂ or 26,27-Dihomo-1α-OH-D₂ | | | |
|---|---|---|---|
| Compound | Dose (pmoles/day) | Ca Transport (S/M) | Serum Calcium (Bone Ca Mobil.) (mg%) |
| -D (Control | 0 | 2.7 ± 0.25 | 4.5 ± 0.04 |
| 1α-OH-D₂ | 50 | 4.7 ± 0.15 | 5.1 ± 0.22 |
| | 125 | 5.3 ± 0.44 | 5.7 ± 0 07 |
| 26,27-Dihomo-24-epi-1α-OH-D₂ | 50 | -- | 3.7 ± 0.13 |
| | 125 | 3.2 ± 0.05 | 4.0 ± 0.30 |
| 26,27-Dihomo-1α-OH-D₂ | 50 | 3.7 ± 0.54 | 4.0 ± 0.18 |
| | 125 | 3.6 ± 0.5 | 3.9 ± 0.18 |

Rats were fed a 0.02% calcium, 0.3% phosphorus vitamin D-deficient diet for 3 weeks and then given the indicated dose intraperitoneally in 0.05 ml of 95% ethanol each day for 7 days. The measurements were made 24 hours after the last dose. There were 6 rats in each group and the data are presented as the mean ± SEM.

**TABLE 4**

| Response of Rachitic Rats to 26,27-Dihomo-1α-OH-D₂ And Its 24-Epimer | | | | | |
|---|---|---|---|---|---|
| Compound | Dose | % Bone Ash | Total mg | Serum P | Line Test |
| -D (Control) | 0 | 22 ± 1.0 | 35 ± 1.3 | 2.3 ± 0.4 | 0 |
| 1α-OH-D₂CEL AL=D>12.5 | 24 ± 1.2 | 39 ± 4.0 | 4.0 ± 0.23 | 4.0 ± 0.4 | |
| | 25.0 | 26 ± 1.5 | 43 ± 5.0 | 4.7 ± 0.13 | 4.4 ± 0.4 |
| | 50.0 | 26 ± 0.7 | 44.2 ± 1.8 | 4.9 ± 0.22 | 5.4 ± 0.38 |
| 26,27-Dihomo-24-epi-1α-OH-D₂ | 12.5 | 18 ± 0.85 | 28 ± 1.2 | 2.8 ± 0.6 | 0 |
| | 25.0 | 18 ± 1.4 | 28 ± 2.6 | 3.1 ± 0.6 | 0 |
| | 50.0 | 18 ± 2.5 | 31 ± 3.5 | 3.6 ± 0.6 | 0 |
| 26,27-Dihomo-1α-OH-D₂ | 12.5 | 21 ± 1.2 | 35 ± 1.3 | 2.9 ± 0.2 | 0 |
| | 25.0 | 18 ± 1.4 | 29 ± 2.3 | 3.4 ± 0.31 | 0 |
| | 50.0 | 21 ± 0.7 | 35 ± 1.9 | 4.2 ± 0.2 | 0 |

The rats were fed for 3 weeks on a high calcium (1.2%), low phosphorus (0.1%) diet, and then given the indicated dose in 0.05 ml 95% ethanol each day for 7 days. The measurements were made 24 hours after the last dose.

Line test is degree of mineralization of rachitic epiphyseal plate (see U. S. Pharmacopeia, 1955). Femurs were removed, cleaned and defatted with ethanol for 24 hours and CHCl₃ for 24 hours using a Soxhlet extractor. After dry weight measurement, the bones were ashed in a muffle furnace at 600°F for 24 hours. The % ash or total ash per femur was recorded. There were 6 rats per group.

**TABLE 5**

| 26,27-Dihomo-24-epi-1,25-(OH)₂D₃ | | |
|---|---|---|
| | NBT | Phago |
| 1 x 10⁻⁷ | 86 ± 4 | 88 ± 2 |
| 5 x 10⁻⁸ | 75 ± 3 | 73 ± 2 |
| 1 x 10⁻⁸ | 62 ± 1 | 63 ± 2 |
| 1 x 10⁻⁹ | 36 ± 3 | 35 ± 2 |

| 26,27-Dihomo-24-Epi-1α-OH-D₂ | | |
|---|---|---|
| 5 x 10⁻⁷ | 36 ± 3 | 43 ± 4 |
| 2.5 x 10⁻⁷ | 12 ± 3 | 13 ± 2 |
| 1 x 10⁻⁷ | 6 ± 1 | 7 ± 2 |
| 5 x 10⁻⁸ | 5 ± 2 | 5 |
| 1 x 10⁻⁶ | 5 ± 1 | 5 |

| 26,27-Dihomo-1α-OH-D₂ | | |
|---|---|---|
| 1 x 10⁻⁶ | 82 ± 3 | 83 ± 4 |
| 5 x 10⁻⁷ | 76 ± 2 | 68 ± 4 |
| 1 x 10⁻⁷ | 61 ± 3 | 57 ± 3 |
| 1 x 10⁻⁸ | 20 ± 3 | 23 ± 2 |

Thus the preceding assays demonstrate that the new compounds, 26,27-dihomo-1α-hydroxyvitamin D₂ and 26,27-dihomo-1α-hydroxy-24-epi-vitamin D₂, exhibit a very distinct and unique spectrum of activities. The 26,27-homologated epi-analog actually diminishes percent bone ash and total bone ash, indicating that it is antagonizing any remaining endogenous vitamin D found in the animals. In support of this, the epi-compound diminishes serum calcium below control values in the case of the animals maintained on a low calcium diet. These two lines of evidence demonstrate the antagonistic nature of this compound which is unique in the art. The new 26,27 homologated epi compound, in particular, therefore, represents a valuable addition to the repertoire of useful therapeutic agents, and may be applied advantageously in cases of hypercalcemia of diverse origins.

For treatment purposes, the novel compound of this invention may be formulated as a solution in innocuous solvents, or as an emulsion, suspension or dispersion in suitable solvents or carriers, or as pills, tablets or capsules, together with solid carriers, according to conventional methods known in the art. The compounds are advantageously administered by injection, or by intravenous infusion of suitable sterile solutions, or in the form of liquid or solid doses via the alimentary canal. Doses of from 1 »g to 50 »g per day, particularly of 1α-hydroxy-24-epi-vitamin D₂, are appropriate for treatment purposes, such doses being adjusted according to the disease to be treated and the response of the subject as is well understood in the art. Since the new epi compound exhibits specificity of action, it is suitably administered alone, in situations where only calcium transport stimulation is desired, or together with graded doses of another active vitamin D compound -- e.g. 1α-hydroxyvitamin D₂ or D₃, or 1α,25-dihydroxyvitamin D₃ -- in situations where some degree of bone mineral mobilization (together with calcium transport stimulation) is found to be advantageous.

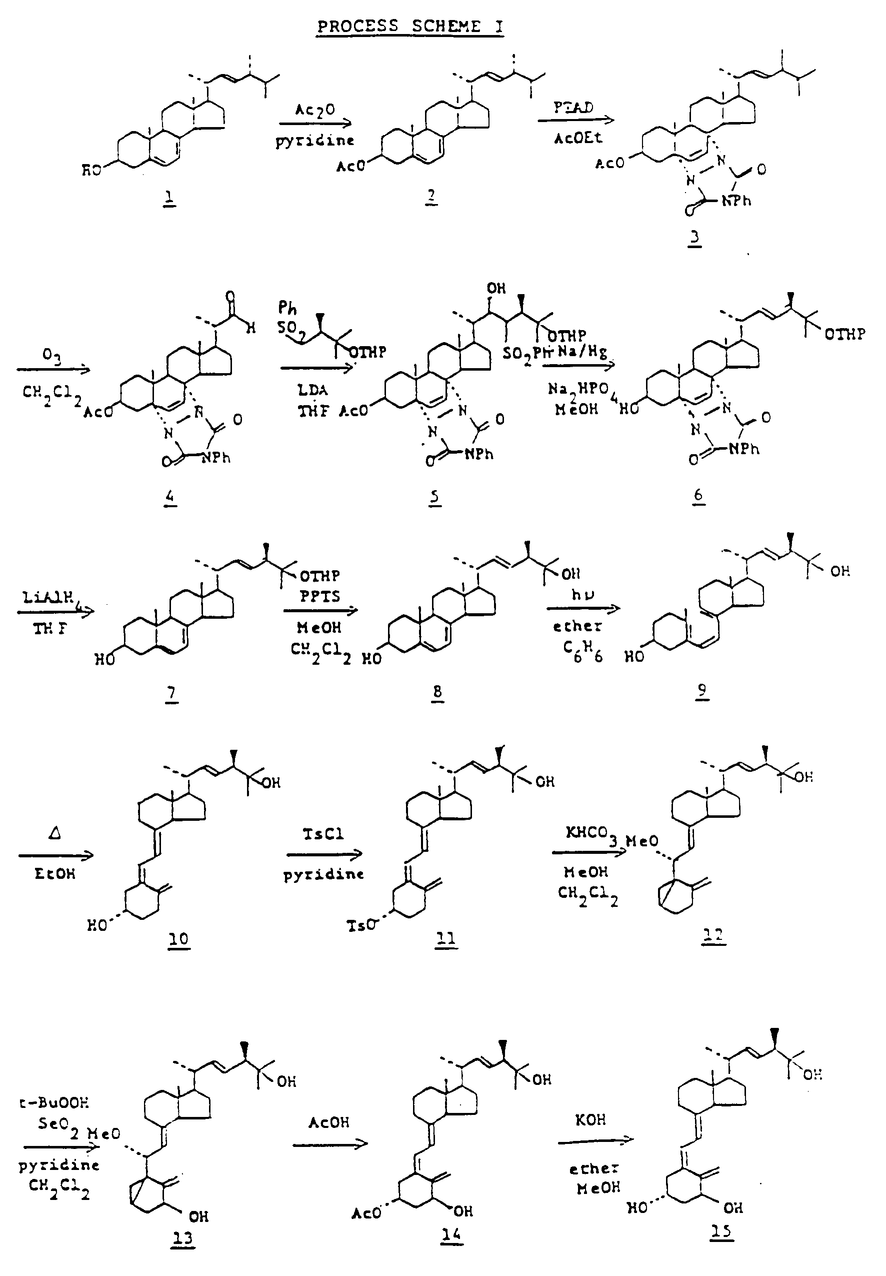

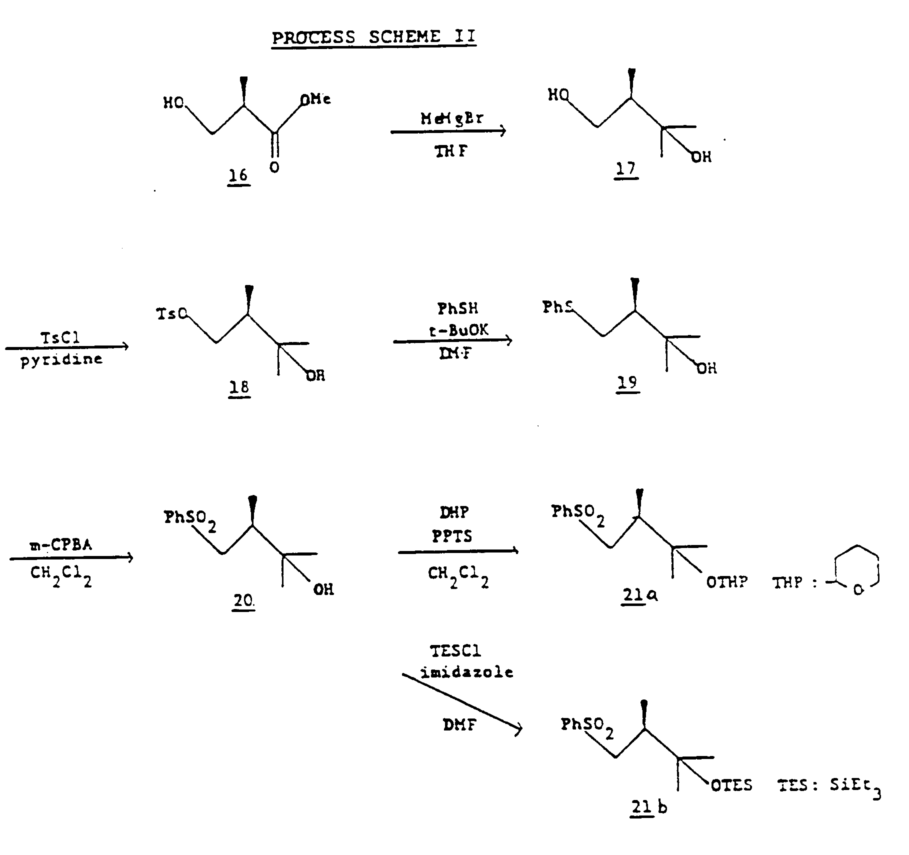

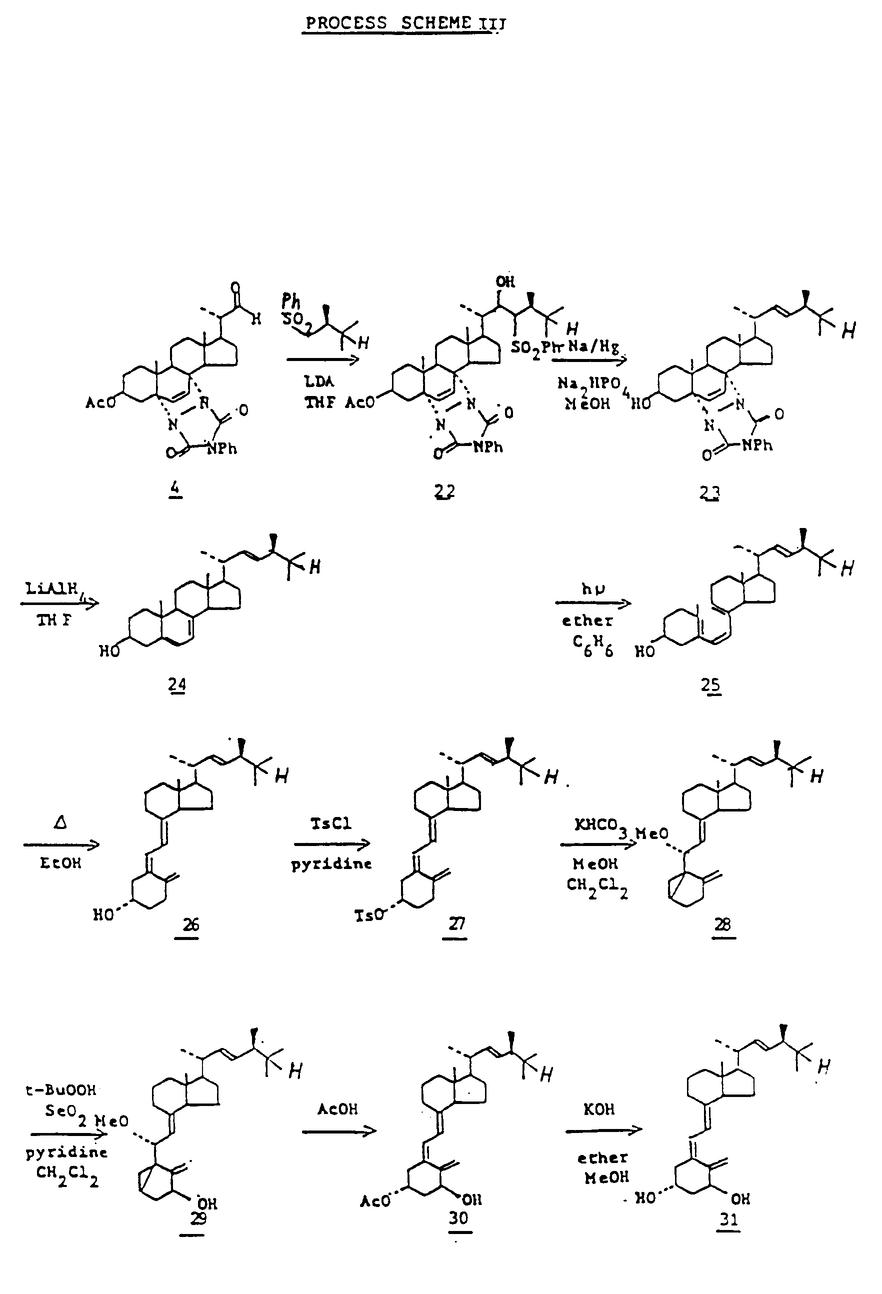

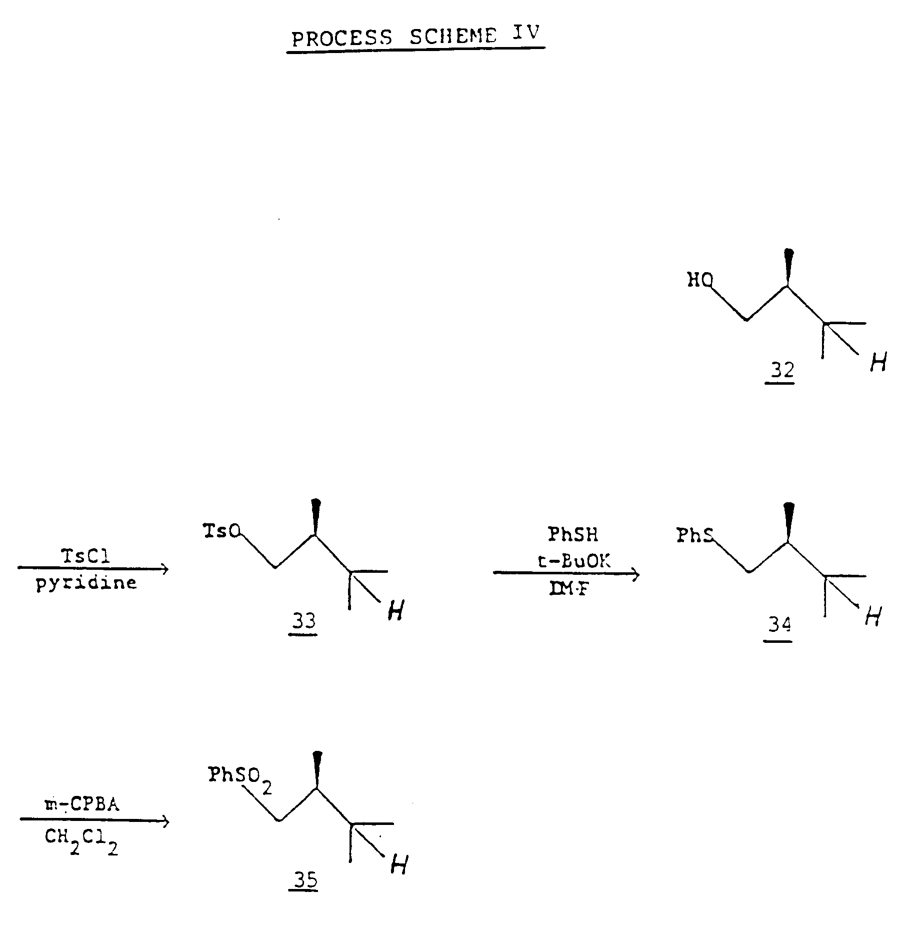

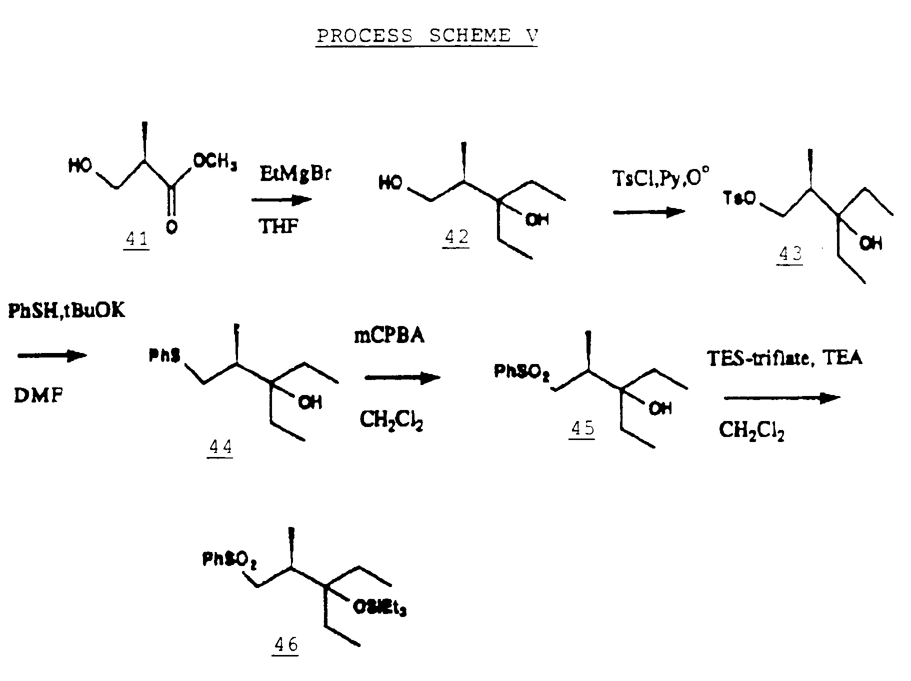

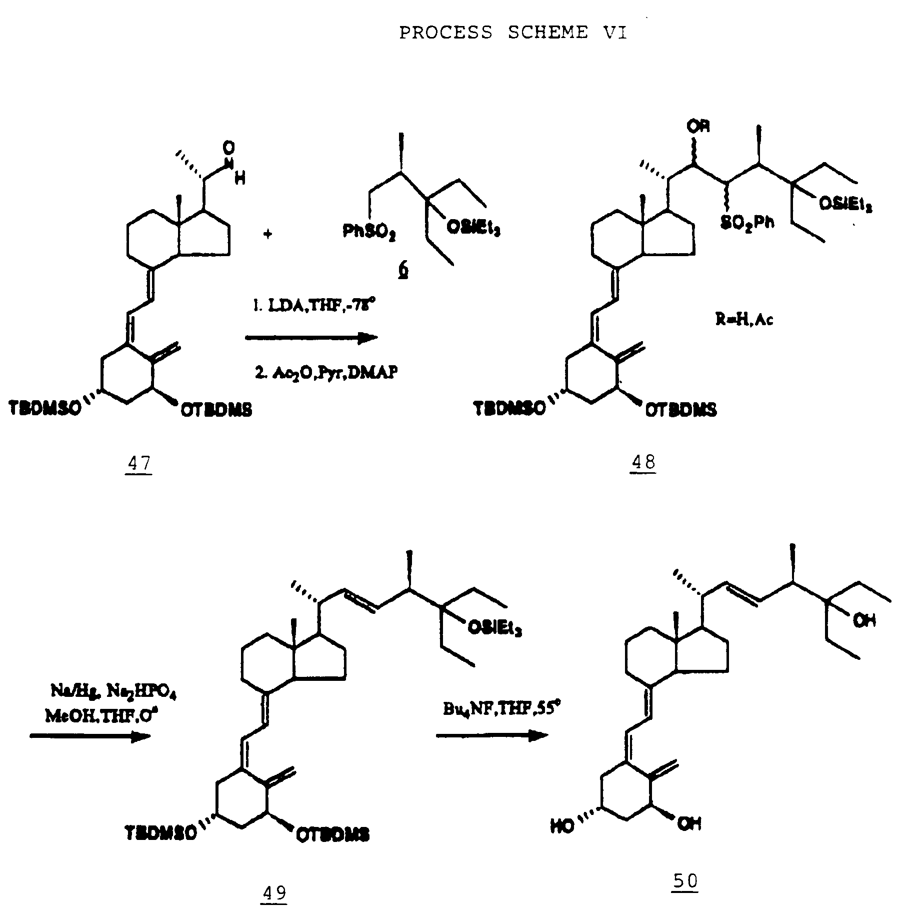

## Claims (Claims for the following Contracting State(s): AT, BE, CH, DE, DK, FR, GB, IT, LI, LU, NL, SE)

1. A vitamin D compound having the formula 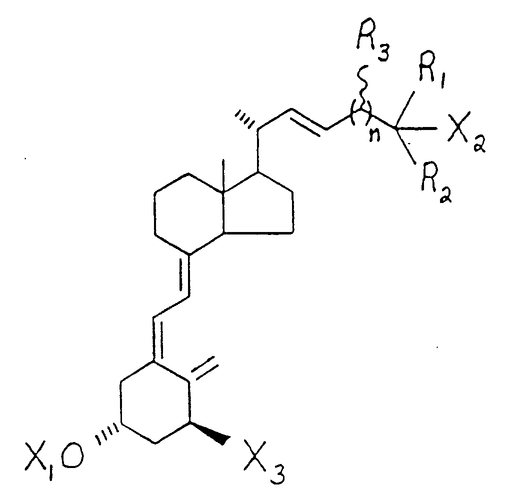 where the configuration about carbon 24 may be R or S and wherein n is an integer having a value of from 1 to 5, X₁ is hydrogen or a hydroxy protecting group, X₂ is hydrogen, hydroxy, or protected hydroxy, X₃ is hydrogen, hydroxy, or protected hydroxy, R₃ is alkyl, hydroxy, protected hydroxy, hydrogen or fluorine, and wherein R₁ and R₂, which may be the same or different, are each an alkyl or aryl group, with the proviso that when X₁ is hydrogen, X₂ is hydrogen or hydroxy, X₃ is hydrogen or hydroxy and n is 1, R₁, and R₂ cannot both be methyl and R₃ cannot be hydrogen or methyl.

2. A compound of claim 1 wherein X₁ and X₂ are both hydrogen, and X₃ is hydroxy.

3. A compound of claim 1 wherein X₁ and X₃ are both hydrogen, and X₂ is hydroxy.

4. A compound of claim 1 wherein X₁ is hydrogen, and X₂ and X₃ are both hydroxy.

5. 26,27-dihomo-1α-hydroxyvitamin D₂.

6. 26,27-dihomo-1α-hydroxy-24-epi-vitamin D₂.

7. 26,27-dihomo-1α,25-dihydroxy-24-epi-vitamin D₂.

8. A pharmaceutical composition comprising a compound as claimed in any one of the preceding claims together with a pharmaceutically acceptable excipient.

9. The pharmaceutical composition of claim 8, wherein said compound is 26,27-dihomo-1α-hydroxyvitamin D₂.

10. The pharmaceutical composition of claim 8 wherein said compound is 26,27-dihomo-1α,hydroxy-24-epi-vitamin D₂.

11. The pharmaceutical composition of claim 8, wherein said compound is 26,27-dihomo-1α,25-dihydroxy-24-epi-vitamin D₂.

## Claims (Claims for the following Contracting State(s): ES, GR)

1. A process for preparing a vitamin D compound having the formula 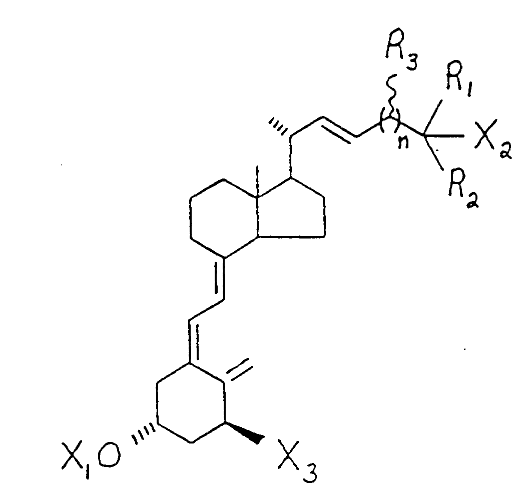 where the configuration about carbon 24 may be R or S and wherein n is an integer having a value of from 1 to 5, X₁ is hydrogen or a hydroxy protecting group, X₂ is hydrogen, hydroxy, or protected hydroxy, X₃ is hydrogen, hydroxy, or protected hydroxy, R₃ is alkyl, hydroxy, protected hydroxy, hydrogen or fluorine, and wherein R₁ and R₂, which may be the same or different, are each an alkyl or aryl group, with the proviso that when X₁ is hydrogen, X₂ is hydrogen or hydroxy, X₃ is hydrogen or hydroxy and n is 1, R₁, and R₂ cannot both be methyl and R₃ cannot be hydrogen or methyl, which comprises solvolysing a cyclovitamin derivative of the formula: 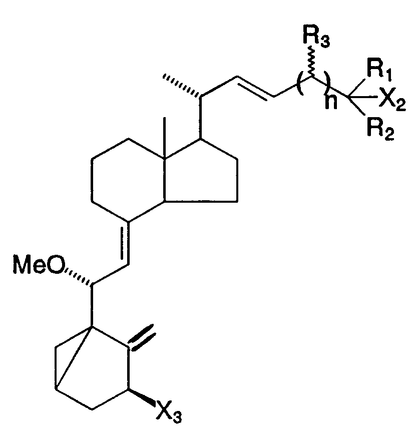 in the presence of a low molecular weight carboxylic acid.

2. A process according to claim 1 wherein the acid is acetic acid.

3. A process for preparing a vitamin D compound having the formula 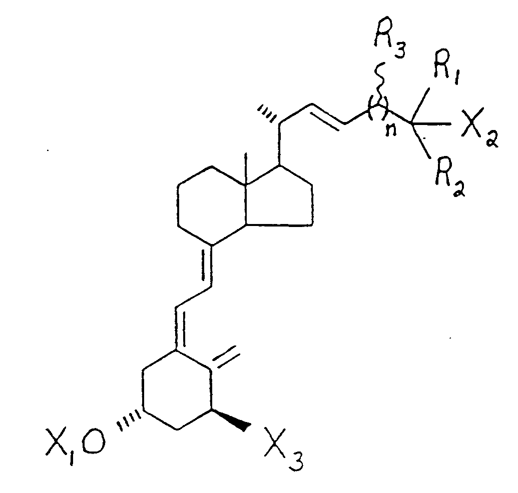 there the configuration about carbon 24 may be R or S and wherein n is an integer having a value of from 1 to 5, X₁ is hydrogen or a hydroxy protecting group, X₂ is hydrogen, hydroxy, or protected hydroxy, X₃ is hydrogen, hydroxy, or protected hydroxy, R₃ is alkyl, hydroxy, protected hydroxy, hydrogen or flourine, and wherein R₁ and R₂ which may be the same or different, are each an alkyl or aryl group, with the proviso that when X₁ is hydrogen, X₂ is hydrogen or hydroxy, X₃ is hydrogen or hydroxy and n is 1, R₁, and R₂ cannot both be methyl and R₃ cannot be hydrogen or methyl, which comprises reducing a compound of the formula: 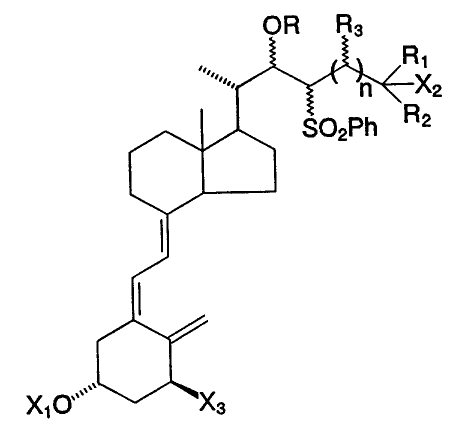 wherein R is hydrogen or acetyl with a metal amalgam.

4. A process according to claim 3 wherein the metal amalgam is sodium amalgam.

5. A process according to any one of claims 1 to 4 wherein X₁ and X₂ are both hydrogen, and X₃ is hydroxy.

6. A process according to any one of claims 1 to 4 wherein X₁ and X₃ are both hydrogen, and X₂ is hydroxy.

7. A process according to any one of claims 1 to 4 wherein X₁ is hydrogen, and X₂ and X₃ are both hydroxy.

8. A process according to any one of claims 1 to 4 for preparing 26,27-dihomo-1α-hydroxyvitamin D₂.

9. A process according to any one of claims 1 to 4 for preparing 26,27-dihomo-1α-hydroxy-24-epi-vitamin D₂.

10. A process according to any one of claims 1 to 4 for preparing 26,27-dihomo-1α,25-dihydroxy-24-epi-vitamin D₂.

11. A process for preparing a pharmaceutical composition which comprises mixing a compound as defined in any one of the preceding claims with a pharmaceutically acceptable excipient.

12. The process of claim 11 wherein said compound is 26,27-dihomo-1α-hydroxyvitamin D₂.

13. The process of claim 11 wherein said compound is 26,27-dihomo-1α,hydroxy-24-epi-vitamin D₂.

14. The process of claim 11, wherein said compound is 26,27-dihomo-1α,25-dihydroxy-24-epi-vitamin D₂.

## Patentansprüche (Patentansprüche für folgende(n) Vertragsstaat(en): AT, BE, CH, DE, DK, FR, GB, IT, LI, LU, NL, SE)

1. Vitamin D-Verbindung der Formel 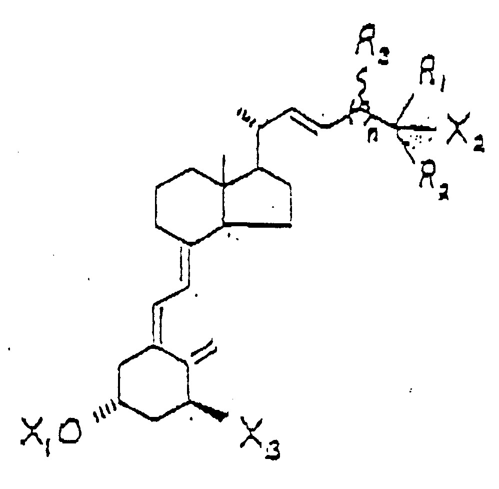 worin die Konfiguration am Kohlenstoffatom 24 R oder S sein kann, und worin n eine ganze Zahl von 1 bis 5 ist, X₁ Wasserstoff oder eine Hydroxy-Schutzgruppe ist, X₂ Wasserstoff, Hydroxy, oder geschütztes Hydroxy ist, X₃ Wasserstoff, Hydroxy oder geschütztes Hydroxy ist, R₃ Alkyl, Hydroxy, geschütztes Hydroxy, Wasserstoff oder Fluor ist, und worin R₁ und R₂, die gleich oder verschieden sein können, jeweils eine Alkyl- oder Arylgruppe bedeuten, mit der Maßgabe, daß, wenn X₁ Wasserstoff, X₂ Wasserstoff oder Hydroxy, X₃ Wasserstoff oder Hydroxy und n 1 ist, R₁ und R₂ nicht Methyl, und R₃ nicht Wasserstoff oder Methyl sind.

2. Verbindung nach Anspruch 1, dadurch gekennzeichnet, daß X₁ und X₂ beide Wasserstoff sind, und X₃ Hydroxy ist.

3. Verbindung nach Anspruch 1, dadurch gekennzeichnet, daß X₁ und X₃ beide Wasserstoff sind, und X₂ Hydroxy ist.

4. Verbindung nach Anspruch 1, dadurch gekennzeichnet, daß X₁ Wasserstoff ist und X₂ und X₃ beide Hydroxy sind.

5. 26,27-Dihomo-1α-hydrovitamin D₂.

6. 26,27-Dihomo-1α-hydroxy-24-epi-Vitamin D₂.

7. 26,27-Dihomo-1α,25-dihydroxy-24-epi-Vitamin D₂.

8. Pharmazeutische Zusammensetzung, dadurch gekennzeichnet, daß sie eine in einem der vorhergehenden Ansprüche beanspruchte Verbindung zusammen mit einem pharmazeutisch annehmbaren Träger umfaßt.

9. Pharmazeutische Zusammensetzung nach Anspruch 8, dadurch gekennzeichnet, daß die Verbindung 26,27-Dihomo-1α-hydroxyvitamin D₂ ist.

10. Pharmazeutische Zusammensetzung nach Anspruch 8, dadurch gekennzeichnet, daß die Verbindung 26, 27-Dihomo-1α,hydroxy-24-epi-Vitamin D₂ ist.

11. Pharmazeutische Zusammensetzung nach Anspruch 8, dadurch gekennzeichnet, daß die Verbindung 26,27-Dihomo-1α,25-dihydroxy-24-epi-Vitamin D₂ ist.

## Patentansprüche (Patentansprüche für folgende(n) Vertragsstaat(en): ES, GR)

1. Verfahren zur Herstellung einer Vitamin D-Verbindung der Formel 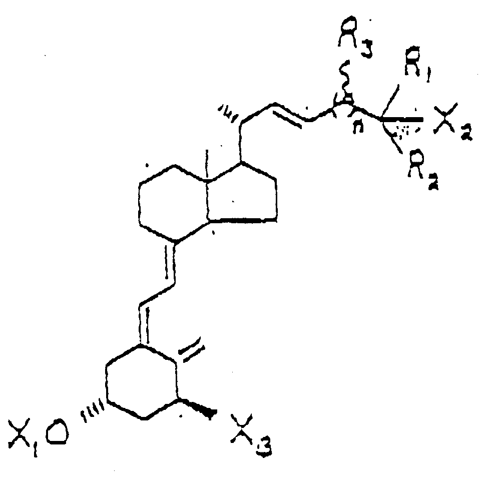 worin die Konfiguration am Kohlenstoffatom 24 R oder S sein kann, und worin n eine ganze Zahl von 1 bis 5 ist, X₁ Wasserstoff oder eine Hydroxy-Schutzgruppe ist, X₂ Wasserstoff, Hydroxy, oder geschütztes Hydroxy ist, X₃ Wasserstoff, Hydroxy oder geschütztes Hydroxy ist, R₃ Alkyl, Hydroxy, geschütztes Hydroxy, Wasserstoff oder Fluor ist, und worin R₁ und R₂, die gleich oder verschieden sein können, jeweils eine Alkyl- oder Arylgruppe bedeuten, mit der Maßgabe, daß, wenn X₁ Wasserstoff, X₂ Wasserstoff oder Hydroxy, X₃ Wasserstoff oder Hydroxy und n 1 ist, R₁ und R₂ nicht Methyl, und R₃ nicht Wasserstoff oder Methyl sind, dadurch gekennzeichnet, daß man ein Cyclovitamin-Derivat der Formel 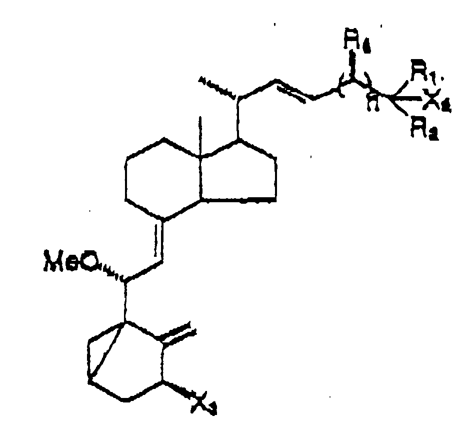 in Gegenwart einer Carbonsäure mit niedrigem Molekulargewicht einer Solvolyse unterwirft.

2. Verfahren nach Anspruch 1, dadurch gekennzeichnet, daß die Säure Essigsäure ist.

3. Verfahren zur Herstellung einer Vitamin D-Verbindung der Formel 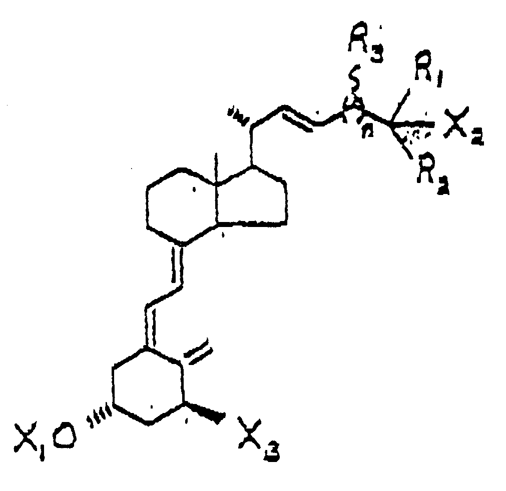 worin die Konfiguration am Kohlenstoffatom 24 R oder S sein kann, und worin n eine ganze Zahl von 1 bis 5 ist, X₁ Wasserstoff oder eine Hydroxy-Schutzgruppe ist, X₂ Wasserstoff, Hydroxy, oder geschütztes Hydroxy ist, X₃ Wasserstoff, Hydroxy oder geschütztes Hydroxy ist, R₃ Alkyl, Hydroxy, geschütztes Hydroxy, Wasserstoff oder Fluor ist, und worin R₁ und R₂, die gleich oder verschieden sein können, jeweils eine Alkyl- oder Arylgruppe bedeuten, mit der Maßgabe, daß, wenn X₁ Wasserstoff, X₂ Wasserstoff oder Hydroxy, X₃ Wasserstoff oder Hydroxy und n 1 ist, R₁ und R₂ nicht Methyl, und R₃ nicht Wasserstoff oder Methyl sind, dadurch gekennzeichnet, daß man eine Verbindung der Formel 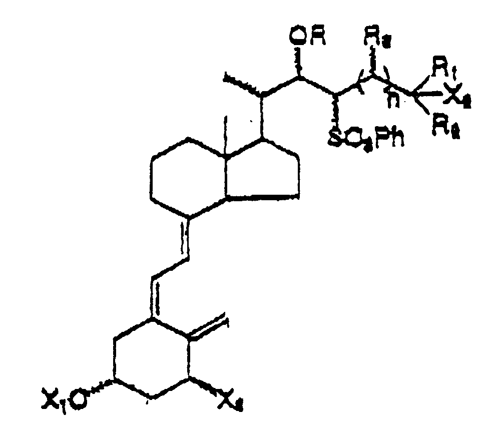 worin R Wasserstoff oder Acetyl bedeutet, mit einem Metallamalgam reduziert.

4. Verfahren nach Anspruch 3, dadurch gekennzeichnet, daß das Metallamalgam Natriumamalgam ist.

5. Verfahren nach einem der Ansprüche 1 bis 4, dadurch gekennzeichnet, daß X₁ und X₂ beide Wasserstoff sind, und X₃ Hydroxy ist.

6. Verfahren nach einem der Ansprüche 1 bis 4, dadurch gekennzeichnet, daß X₁ und X₃ beide Wasserstoff sind, und X₂ Hydroxy ist.

7. Verfahren nach einem der Ansprüche 1 bis 4, dadurch gekennzeichnet, daß X₁ Wasserstoff ist und X₂ und X₃ beide Hydroxy sind.

8. Verfahren nach einem der Ansprüche 1 bis 4, dadurch gekennzeichnet, daß man 26,27-Dihomo-1α-hydroxyvitamin D₂ herstellt.

9. Verfahren nach einem der Ansprüche 1 bis 4, dadurch gekennzeichnet, daß man 26,27-Dihomo-1α-hydroxy-24-epi-Vitamin D₂ herstellt.

10. Verfahren nach einem der Ansprüche 1 bis 4, dadurch gekennzeichnet, daß man 26,27-Dihomo-1α,25-dihydroxy-24-epi-Vitamin D₃ herstellt.

11. Verfahren zur Herstellung einer pharmazeutischen Zusammensetzung, dadurch gekennzeichnet, daß man eine in einem der vorhergehenden Ansprüche definierten Verbindung zusammen mit einem pharmazeutisch annehmbaren Träger mischt.

12. Verfahren nach Anspruch 11, dadurch gekennzeichnet, daß die Verbindung 26,27-Dihomo-1α-hydroxyvitamin D₂ ist.

13. Verfahren nach Anspruch 11, dadurch gekennzeichnet, daß die Verbindung 26,27-Dihomo-1α-hydroxy-24-epi-Vitamin D₂ ist.

14. Verfahren nach Anspruch 11, dadurch gekennzeichnet, daß die Verbindung 26,27-Dihomo-1α,25-dihydroxy-24-epi-Vitamin D₂ ist.

## Revendications (Revendications pour l'(les) Etat(s) contractant(s) suivant(s): AT, BE, CH, DE, DK, FR, GB, IT, LI, LU, NL, SE)

1. Composé de type vitamine D de formule 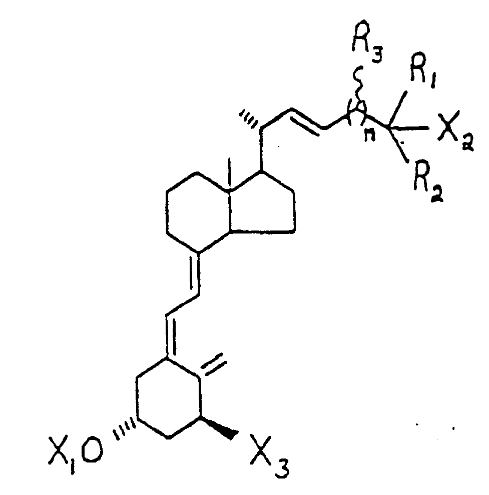 dans laquelle la configuration de l'atome de carbone C-24 peut être R ou S et dans laquelle n est un nombre entier valant entre 1 et 5, X₁ représente un atome d'hydrogène ou un groupe hydroxyle protégé, X₂ représente un atome d'hydrogène, un groupe hydroxyle ou un groupe hydroxyle protégé, X₃ représente un atome d'hydrogène, un groupe hydroxyle ou un groupe hydroxyle protégé, R₃ représente un groupe alkyle, hydroxyle, hydroxyle protégé, un atome d'hydrogène ou de fluor, et dans laquelle R₁ et R₂, identiques ou différents, représentent chacun un groupe alkyle ou aryle, à condition que, lorsque X₁ est un atome d'hydrogène, X₂ soit un atome d'hydrogène ou un groupe hydroxyle, X₃ soit un atome d'hydrogène ou un groupe hydroxyle et n vaille 1, R₁ et R₂ ne puissent pas être tous les deux un groupe méthyle et R₃ ne puisse pas être un atome d'hydrogène ou un groupe méthyle.

2. Composé conforme à la revendication 1 dans lequel X₁ et X₂ représentent tous les deux un atome d'hydrogène et X₃ un groupe hydroxyle.

3. Composé conforme à la revendication 1 dans lequel X₁ et X₃ représentent tous les deux un atome d'hydrogène et X₂ un groupe hydroxyle.

4. Composé conforme à la revendication 1 dans lequel X₁ est un atome d'hydrogène et X₂ et X₃ représentent tous les deux un groupe hydroxyle.

5. 26,27-dihomo-1α-hydroxyvitamine D₂.

6. 26,27-dihomo-1α-hydroxy-24-épi-vitamine D₂.

7. 26,27-dihomo-1α,25-dihydroxy-24-épi-vitamine D₂.

8. Composition pharmaceutique contenant un composé conforme à une quelconque des revendications précédentes, ensemble avec un excipient pharmaceutiquement acceptable.

9. Composition pharmaceutique conforme à la revendication 8 dans laquelle ledit composé est la 26,27-dihomo-1α-hydroxyvitamine D₂.

10. Composition pharmaceutique conforme à la revendication 8 dans laquelle ledit composé est la 26,27-dihomo-1α-hydroxy-24-épi-vitamine D₂.

11. Composition pharmaceutique conforme à la revendication 8 dans laquelle ledit composé est la 26,27-dihomo-1α,25-dihydroxy-24-épi-vitamine D₂.

## Revendications (Revendications pour l'(les) Etat(s) contractant(s) suivant(s): ES, GR)

1. Procédé de préparation d'un composé de type vitamine D de formule 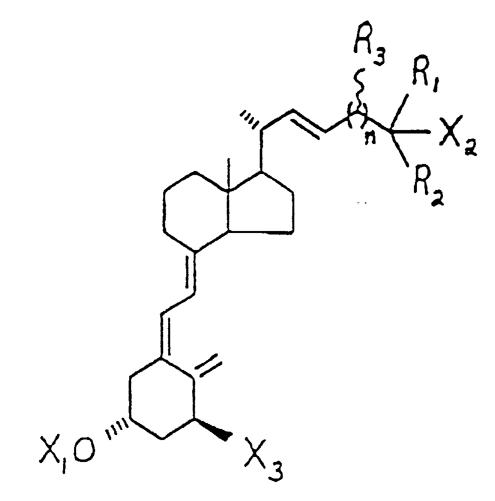 dans laquelle la configuration de l'atome de carbone C-24 peut être R ou S et dans laquelle n est un nombre entier valant entre 1 et 5, X₁ représente un atome d'hydrogène ou un groupe hydroxyle protégé, X₂ représente un atome d'hydrogène, un groupe hydroxyle ou un groupe hydroxyle protégé, X₃ représente un atome d'hydrogène, un groupe hydroxyle ou un groupe hydroxyle protégé, R₃ représente un groupe alkyle, hydroxyle, hydroxyle protégé, un atome d'hydrogène ou de fluor, et dans laquelle R₁ et R₂, identiques ou différents, représentent chacun un groupe alkyle ou aryle, à condition que, lorsque X₁ est un atome d'hydrogène, X₂ soit un atome d'hydrogène ou un groupe hydroxyle, X₃ soit un atome d'hydrogène ou un groupe hydroxyle et n vaille 1, R₁ et R₂ ne puissent pas être tous les deux un groupe méthyle et R₃ ne puisse pas être un atome d'hydrogène ou un groupe méthyle, lequel procédé comprend la solvolyse d'un dérivé de type cyclovitame de formule : 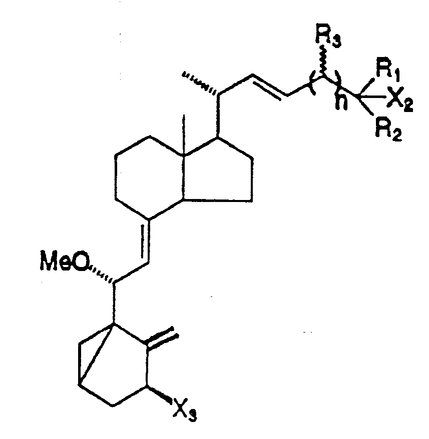 en présence d'un acide carboxylique de faible masse moléculaire.

2. Procédé conforme à la revendication 1 dans lequel l'acide est l'acide acétique.

3. Procédé de préparation d'un composé de type vitamine D de formule 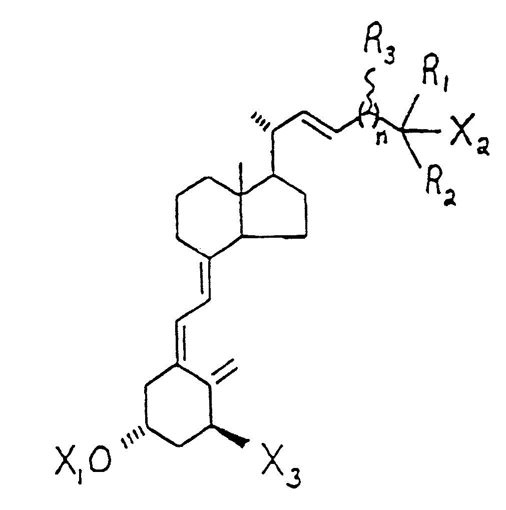 dans laquelle la configuration de l'atome de carbone C-24 peut être R ou S et dans laquelle n est un nombre entier valant entre 1 et 5, X₁ représente un atome d'hydrogène ou un groupe hydroxyle protégé, X₂ représente un atome d'hydrogène, un groupe hydroxyle ou un groupe hydroxyle protégé, X₃ représente un atome d'hydrogène, un groupe hydroxyle ou un groupe hydroxyle protégé, R₃ représente un groupe alkyle, hydroxyle, hydroxyle protégé, un atome d'hydrogène ou de fluor, et dans laquelle R₁ et R₂, identiques ou différents, représentent chacun un groupe alkyle ou aryle, à condition que, lorsque X₁ est un atome d'hydrogène, X₂ soit un atome d'hydrogène ou un groupe hydroxyle, X₃ soit un atome d'hydrogène ou un groupe hydroxyle et n vaille 1, R₁ et R₂ ne puissent pas être tous les deux un groupe méthyle et R₃ ne puisse pas être un atome d'hydrogène ou un groupe méthyle, lequel procédé comprend la réduction d'un composé de formule : 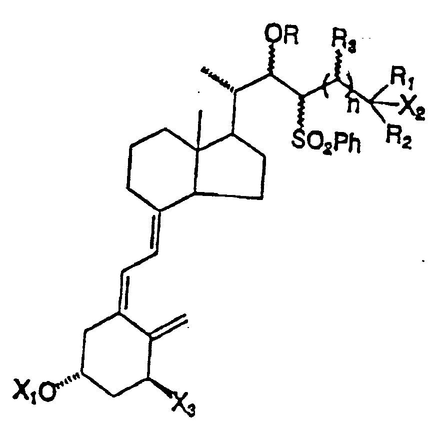 dans laquelle R représente un atome d'hydrogène ou un résidu acétyle par un amalgame métallique.

4. Procédé conforme à la revendication 3 dans lequel l'amalgame métallique est un amalgame de sodium.

5. Procédé conforme à une quelconque des revendications 1 à 4 dans lequel X₁ et X₂ représentent tous les deux un atome d'hydrogène et X₃ un groupe hydroxyle.

6. Procédé conforme à une quelconque des revendications 1 à 4 dans lequel X₁ et X₃ représentent tous les deux un atome d'hydrogène et X₂ un groupe hydroxyle.

7. Procédé conforme à une quelconque des revendications 1 à 4 dans lequel X₁ est un atome d'hydrogène et X₂ et X₃ représentent tous les deux un groupe hydroxyle.

8. Procédé conforme à une quelconque des revendications 1 à 4 pour préparer la 26,27-dihomo-1α-hydroxyvitamine D₂.

9. Procédé conforme à une quelconque des revendications 1 à 4 pour préparer la 26,27-dihomo-1α-hydroxy-24-épi-vitamine D₂.

10. Procédé conforme à une quelconque des revendications 1 à 4 pour préparer la 26,27-dihomo-1α,25-dihydroxy-24-épi-vitamine D₂.

11. Procédé de préparation d'une composition pharmaceutique consistant à mélanger un composé défini par une quelconque des revendications précédentes avec un excipient pharmaceutiquement acceptable.

12. Procédé conforme à la revendication 11 dans lequel ledit composé est la 26,27-dihomo-1α-hydroxyvitamine D₂.

13. Procédé conforme à la revendication 11 dans lequel ledit composé est la 26,27-dihomo-1α-hydroxy-24-épi-vitamine D₂.

14. Procédé conforme à la revendication 11 dans lequel ledit composé est la 26,27-dihomo-1α,25-dihydroxy-24-épi-vitamine D₂.
